Europäisches Patentamt
European Patent Office
Office européen des brevets

(19) (11) Publication number: **0 269 363 A2**

# (12) EUROPEAN PATENT APPLICATION

(21) Application number: 87310203.2

(22) Date of filing: 19.11.87

(51) Int. Cl.[4]: C07C 93/06 , C07C 93/02 , C07C 149/42 , C07C 149/26 , C07D 295/08 , C07D 211/14 , C07D 265/30 , C07D 215/04 , A01N 33/08 , A01N 33/10 , A01N 43/40

(30) Priority: 21.11.86 DK 5593/86
15.07.87 DK 3686/87

(43) Date of publication of application:
01.06.88 Bulletin 88/22

(84) Designated Contracting States:
AT BE CH DE ES FR GB GR IT LI LU NL SE

(71) Applicant: A/S CHEMINOVA
P.O. Box 9
DK-7620 Lemvig(DK)

(72) Inventor: Svendsen, Axel
Norrevang 6
DK-7620 Lemvig(DK)
Inventor: Wengel, Anita
Norrevang 6
DK-7620 Lemvig(DK)
Inventor: Kolind-Andersen, Hans
Svanevej 7
DK-7620 Lemvig(DK)
Inventor: Jacobsen, Niels
Kirkensgardvej 33 Vandborg
DK-7620 Lemvig(DK)
Inventor: Klemmensen, Per Dausell
Emilielystvej 9
DK-7620 Lemvig(DK)

(74) Representative: Orr, William McLean et al
URQUHART-DYKES & LORD 5th Floor, Tower
House Merrion Way
Leeds West Yorkshire, LS2 8PA(GB)

## (54) Amino-alkylated hydroxy compounds and their use as fungicides.

(57) Aminoalkylated hydroxy compounds having the formula

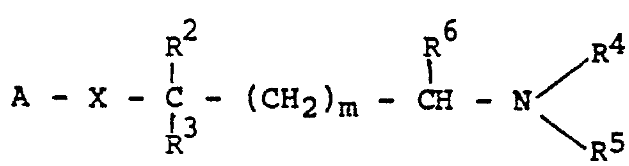

wherein A represents

EP 0 269 363 A2

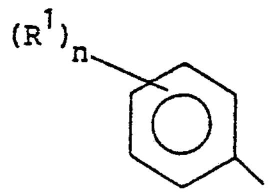

or

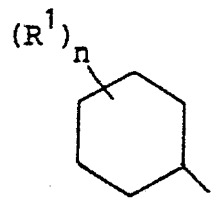

wherein $(R^1)_n$ represents up to four substituents which are the same or different and selected from straight or branched chain, substituted or unsubstituted alkyl having up to 12 carbon atoms, aryl, aryloxy, alkoxy, arylalkyl, haloalkyl, dialkylamino, halogen, alkylthio and nitro, where two adjacent $R^1$-substituents may also together form a saturated or unsaturated ring, and n is 0, 1, 2, 3 or 4, $R^2$ and $R^3$ each independently represents hydrogen or straight or branched chain alkyl having up to 6 carbon atoms, $R^4$ and $R^5$ each independently represents hydrogen, straight or branched chain or cyclic, substituted or unsubstituted alkyl having up to 12 carbon atoms, alkenyl, alkynyl or substituted or unsubstututed arylalkyl, or $R^4$ and $R^5$ when taken together with the nitrogen atom to which they are attached form a saturated or unsaturated, substituted, unsubstituted, aryl-fused or cycloalkyl-fused heterocyclic ring which may optionally contain one or more additional hetero atoms selected from 0 and N, $R^6$ represents hydrogen or lower alkyl, X represents 0 or S, and m is 0, 1 or 2 when A represents substituted or unsubstituted phenyl, and m is 0 or 1, when A represents substituted or unsubstituted cyclohexyl, and quaternary ammonium salts thereof, are prepared in a manner known per se.

The compounds have fungicidal, particularly plant-fungicidal activity and are therefore applicable in fungicidal compositions and in methods for combating fungi, particularly phytopathogenic fungi.

## Amino-alkylated hydroxy compounds and their use as fungicides.

The present invention relates to novel amino-alkylated hydroxy compounds and quaternary ammonium salts thereof, and the invention also relates to the use of said compounds as fungicides.

The novel amino-alkylated hydroxy compounds now provided have the general formula I

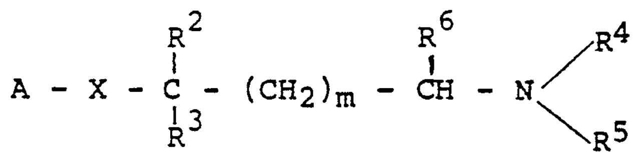

I

wherein A represents

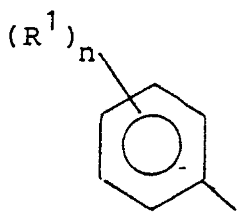

or

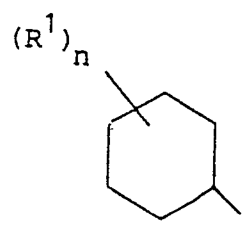

wherein $(R^1)_n$ represents up to four substituents which are the same or different and selected from straight or branched chain, substituted or unsubstituted alkyl having up to 12 carbon atoms, aryl, aryloxy, alkoxy, arylalkyl, haloalkyl, dialkylamino, halogen, alkylthio and nitro, where two adjacent $R^1$-substituents may also together form a saturated or unsaturated ring, and n is 0, 1, 2, 3 or 4,

$R^2$ and $R^3$ each independently represents hydrogen or straight or branched chain alkyl having up to 6 carbon atoms,

$R^4$ and $R^5$ each independently represents hydrogen, straight or branched chain or cyclic, substituted or unsubstituted alkyl having up to 12 carbon atoms, alkenyl, alkynyl or substituted or unsubstituted arylalkyl, or $R^4$ and $R^5$ when taken together with the nitrogen atom to which they are attached form a saturated or unsaturated, substituted, unsubstituted, aryl-fused or cycloalkyl-fused heterocyclic ring which may optionally contain one or more additional hetero atoms selected from 0 and N, $R^6$ represents hydrogen or lower alkyl,

X represents 0 or S, and

m is 0, 1 or 2 when A represents substituted or unsubstituted phenyl, and m is 0 or 1 when A represents substituted or unsubstituted cyclohexyl.

The symbols $R^1$-$R^6$ in the above formula I have preferably, or by way of example, the following meanings:

$R^1$: alkyl is preferably alkyl having 4-10 carbon atoms, more preferably branched chain alkyl having 4-8 carbon atoms,

aryl is preferably phenyl,

aryloxy is preferably phenoxy,

alkoxy is, e.g., methoxy or propoxy,

arylalkyl is, e.g., 1-methyl-1-phenyl-ethyl, alkyl in haloalkyl, dialkylamino and alkylthio is each preferably alkyl having up to 12 carbon atoms, halogen is preferably bromo or iodo, and two adjacent $R^1$-substitutents which together form a saturated or unsaturated ring are preferably fused cyclohexyl or fused phenyl, respectively,

$R^2$ and $R^3$: alkyl is, e.g., methyl,

$R^4$ and $R^5$: straight or branched chain alkyl is preferably alkyl having up to 6 carbon atoms and more preferably up to 3 carbon atoms,

cyclic alkyl is preferably cyclohexyl, alkenyl is preferably allyl,

alkynyl is preferably propargyl,

arylalkyl is preferably benzyl,

the optionally substituted heterocyclic ring is preferably piperidino, mono-or dimethylpiperidino, ethylpiperidino, ethylmethylpiperidino, phenylpiperidino, morpholino, or 2,6-dimethylmorpholino (cis/trans or cis or trans),

aryl in aryl-fused heterocyclic ring is preferably phenyl, and
cycloalkyl in cycloalkyl-fused heterocyclic ring is preferably cyclohexyl, and
$R^6$: lower alkyl is preferably methyl.

The quaternary ammonium salts of the invention are preferably prepared by reaction with alkyl, alkenyl, arylalkyl or alkoxycarbonylmethyl halides, e.g., methyl iodide, allyl iodide, benzyl iodide or ethyl bromoacetate.

It has been found that the novel amino-alkylated hydroxy compounds of formula I and the corresponding quaternary ammonium salts have fungicidal properties.

The active compounds of the invention can be used in practice for combating phytopathogenic fungi as for example Plasmodiophoromycetes, Chytridiomycetes, Zygomycetes, Ascomycetes, Basidiomycetes and Deuteromycetes.

The active substances of the invention can be used, e.g., for combating the plant diseases Puccinia recondita, Puccinia striiformis and other rust diseases on wheat, Puccinia hordei, Puccinia striiformis and other rust diseases on barley and rust on other host plants as for example coffee, apples, vegetables and ornamental plants.
Erysiphe graminis (mildew) on barley and wheat and other true mildew species on various host plants such as Sphaerotheca fuliginea on cucumber, Podosphaera leucotricha on apples and Uncinula necator on vines.
Helminthosporium spp., e.g. Pyrenophora teres (net blotch) on barley.
Rhynchosporium spp. and Pseudocercosporella herpotrichoides on cereals.
Cercospora arachidicola on peanuts and other Cercospora species on for example sugar beet, banana and soya bean.
Botrytis cinerea (grey mold) on tomato, strawberry, vine and other host plants.
Venturia inaequalis (scab) on apples.
Pyricularia oryzae on rice.

The active substances are active in vitro against a broad spectrum of fungi.

The active substances are active as seed dressing agents against Fusarium spp., Septoria spp., Tilletia spp., Ustilago spp., Helminthosporium spp. and Pseudocercosporella herpotrichoides on cereals, Rhizoctonia solani on cotton and Corticium sasakii on rice.

Besides an excellent curative activity the active substances of the invention also show extremely good protective properties as well as systemic activity.

In addition to this some of the compounds are sufficiently volatile to be active in the gaseous phase against fungi on plants.

In accordance with the above the invention also relates to a fungicidal composition, particularly a plant-fungicidal composition, which composition is characterised by containing as an active component at least one compound selected from the amino-alkylated hydroxy compounds of the above formula I and quaternary ammonium salts thereof.

Moreover, the invention relates to the use of an amino-alkylated hydroxy compound of the above formula I and/or a quaternary ammonium salt thereof for combating fungi, particularly phytopathogenic fungi.

Furthermore, the invention relates to a method of combating fungi, particularly phytopathogenic fungi which method is characterised by allowing an amino-alkylated hydroxy compound of the above formula I and/or a quaternary ammonium salt thereof to affect the fungi concerned and/or their biotape.

Furthermore, the invention relates to a process of preparing a fungicidal composition, particularly a plant-fungicidal composition, which process is characterised by mixing an amino-alkylated hydroxy compound of the above formula I and/or a quaternary ammonium salt thereof with one or more agents selected from extending agents, surface active agents and other conventional auxiliary agents.

The amino-alkylated hydroxy compounds of formula I according to the invention and quaternary ammonium salts thereof are exemplified by specific examples of compounds as listed in the table below and can be prepared by any known process for preparing similar compounds, e.g. as illustrated in the following Preparation Examples.

Preparation Example 1

Preparation of 2-(N,N-dipropyl)-aminoethyl 4-(1,1,3,3-tetramethylbutyl)-phenyl ether, compound No. 1.

A mixture of 4-(1,1,3,3-tetramethylbutyl)-phenol (2.06 g, 0.01 mol), N,N-dipropyl-2-chlorethylamine hydrochloride (2.20 g, 0.011 mol), triethylbenzylammonium chloride (0.40 g) and 50% aqueous NaOH (20

g) is heated on an oil bath at 70°C with vigorous stirring. After stirring for 2 hours the mixture is cooled and diluted with 50 ml of water. The mixture is extracted with ether (2 x 50 ml), and the ether phase is separated and dried over $Na_2SO_4$. The solvent is removed in vacuo, and the desired product is isolated from the resulting oil by kugelrohr distillation.

Yield 1.9 g B.p. 230°C/10 mmHg; $n_D^{26}$ = 1.4928.

Preparation Example 2

Preparation of 1-(2,6-dimethyl-4-morpholinyl)-2-methyl-2-propyl 4-(1,1,3,3-tetramethylbutyl)-phenyl ether, compound No. 22.

While distilling-off dimethyl sulphide, borane-methyl sulphide complex (2.51 g, 0.033 mol) is added dropwise to a refluxing stirred solution of the 2,6-dimethylmorpholine amide of 2-methyl-2-(4-(1,1,3,3-tetramethylbutyl)-phenoxy)-propionic acid (4.29 g, 0,011 mol) in 15 ml of dry THF. After cooling the reaction mixture is treated cautiously with HCl (0.025 mol) in 30 ml of methanol and then with aqueous NaOH and ether. The ether phase is dried over $Na_2SO_4$ and evaporated in vacuo, and the desired product is isolated from the resulting oil by kugelrohr distillation.

Yield 3.7 g B.p. 235-245°C/10 mmHg. $n_D^{26}$ = 1.4964.

Preparation Example 3

Preparation of 1-(2,6-dimethyl-4-morpholinyl)-2-propyl 4-(1,1,3,3-tetramethylbutyl)-phenyl ether, compound No. 56.

2-(4-(1,1,3,3-tetramethylbutyl)-phenoxy)-1-propanol methanesulphonate (4.80 g, 0.014 mol), 2,6-dimethylmorpholine (4.03 g, 0.035 mol) and DMF (25 ml) are refluxed while stirring for three hours. After cooling the reaction mixture is treated with ether and several portions of aqueous HCl which is basified with NaOH and extracted with ether. This ether phase is dried over $Na_2SO_4$ and evaporated in vacuo, and the desired product is isolated from the resulting oil by kugelrohr distillation.

Yield 4.04 g. B.p. 215-220°C/8-10 mmHg. $n_D^{26}$ = 1.4988.

Preparation Example 4 Preparation of 2-(1-piperidinyl)-1-1propyl 4-(1,1,3,3-tetramethylbutyl)-phenyl ether, compound No. 119.

1-(4-(1,1,3,3-Tetramethylbutyl)-phenoxy)-2-propanol methanesulphonate (4.13 g,0.012 mol), piperidine (2.98 g, 0.035 mol) and DMF (20 ml) are refluxed while stirring for five hours. After cooling the reaction mixture is treated with ether which is extracted several times with $H_2O$ and then with conc. HCl. The HCl phase is dried over $Na_2SO_4$ and evaporated in vacuo. The desired product is isolated from the resulting oil by kugelrohr distillation.

Yield 2.66 g. B.p. 220-230°C/10-11 mmHg. $n_D^{26}$ = 1.5089.

| | | |
|---|---|---|
| No. 1 | 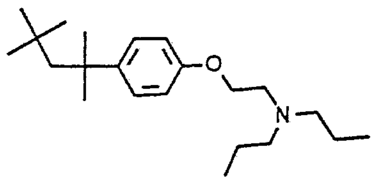 | Refractive Index (26 C) 1.4928 |
| No. 2 | 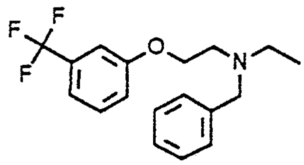 | Refractive Index (26 C) 1.5054 |
| No. 3 | 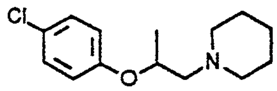 | Refractive Index (26 C) 1.5251 |
| No. 4 | 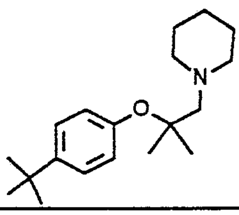 | Melting Point 56-58°C |
| No. 5 | 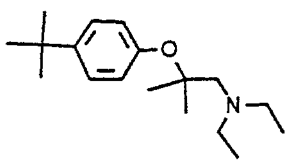 | Refractive Index (26 C) 1.4879 |
| No. 6 | 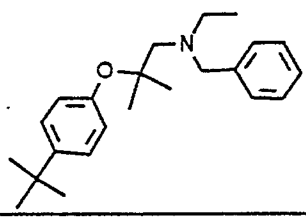 | Refractive Index (26 C) 1.5236 |
| No. 7 | 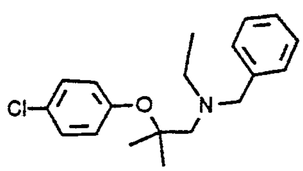 | Refractive Index (26 C) 1.5399 |
| No. 8 | 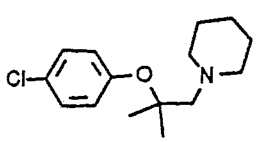 | Refractive Index (26 C) 1.5170 |

No. 9

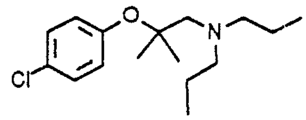

Refractive Index (26 C) 1.4956

---

No. 10

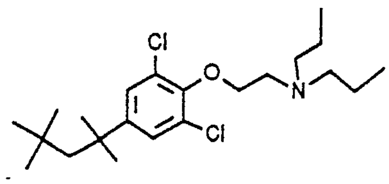

Refractive Index (26 C) 1.5041

---

No. 11

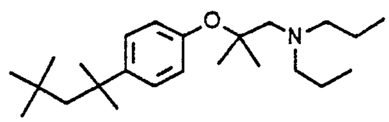

Refractive Index (26 C) 1.4893

---

No. 12

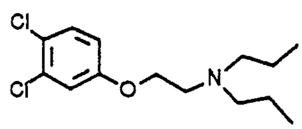

Refractive Index (26 C) 1.5130

---

No. 13

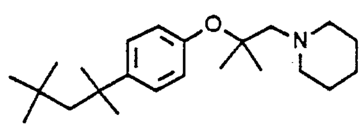

Refractive Index (26 C) 1.5023

---

No. 14

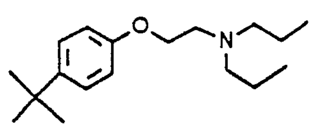

Refractive Index (26 C) 1.4887

---

No. 15

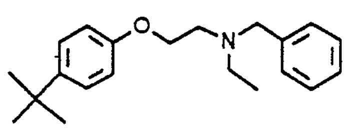

Refractive Index (26 C) 1.5310

---

No. 16

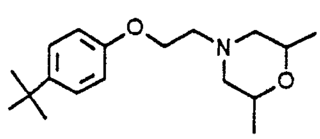

Refractive Index (26 C) 1.5032

---

| | | |
|---|---|---|
| No. 17 | 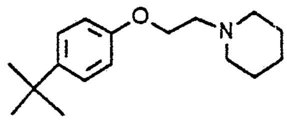 | Refractive Index (26 C) 1.5142 |
| No. 18 | 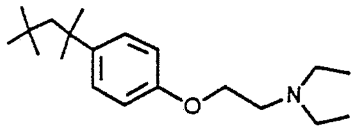 | Refractive Index (26 C) 1.4963 |
| No. 19 | 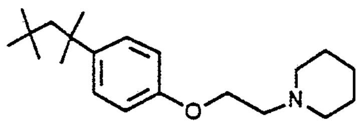 | Refractive Index (26 C) 1.5126 |
| No. 20 | 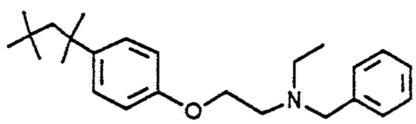 | Refractive Index (26 C) 1.5274 |
| No. 21 | 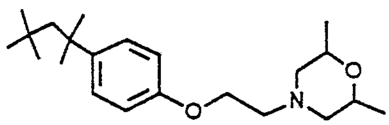 | Refractive Index (26 C) 1.5061 |
| No. 22 | 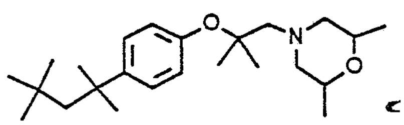 | Refractive Index (26 C) 1.4964 |
| No. 23 | 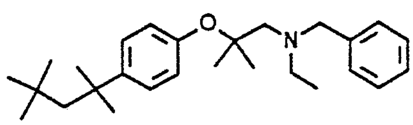 | Refractive Index (26 C) 1.5209 |
| No. 24 | 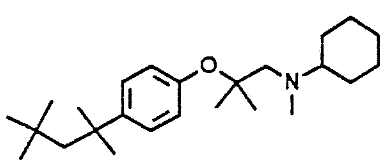 | Refractive Index (26 C) 1.5045 |

| | | |
|---|---|---|
| No. 25 | 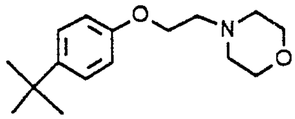 | Refractive Index (26 C) 1.5162 |
| No. 26 | 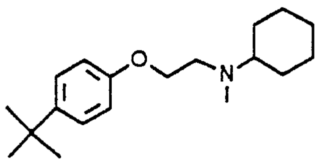 | Refractive Index (26 C) 1.5120 |
| No. 27 | 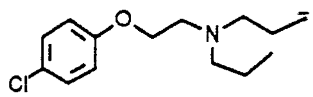 | Refractive Index (26 C) 1.5007 |
| No. 28 | 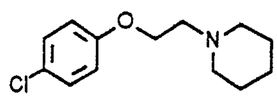 | Refractive Index (26 C) 1.5316 |
| No. 29 | 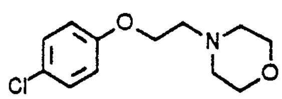 | Refractive Index (26 C) 1.5387 |
| No. 30 | 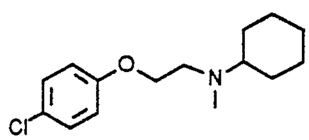 | Refractive Index (26 C) 1.5246 |
| No. 31 | 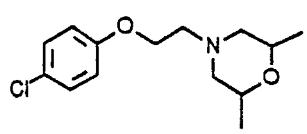 | Refractive Index (26 C) 1.5176 |
| No. 32 | 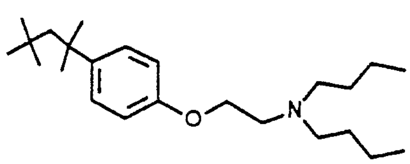 | Refractive Index (26 C) 1.4906 |

No. 33 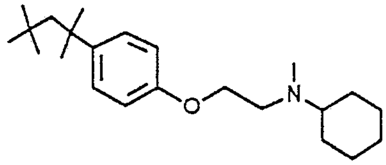 Refractive Index (26 C) 1.5107

No. 34 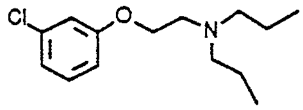 Refractive Index (26 C) 1.5006

No. 35 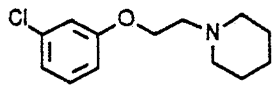 Refractive Index (26 C) 1.5323

No. 36 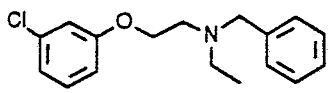 Refractive Index (26 C) 1.5498

No. 37 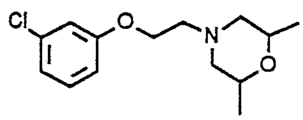 Refractive Index (26 C) 1.5193

No. 38 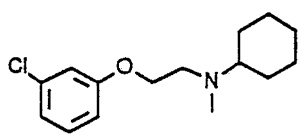 Refractive Index (26 C) 1.5254

No. 39 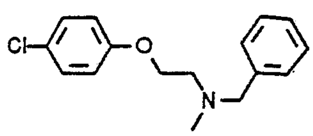 Refractive Index (26 C) 1.5591

No. 40 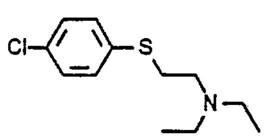 Refractive Index (26 C) 1.5573

| | | |
|---|---|---|
| No. 41 | 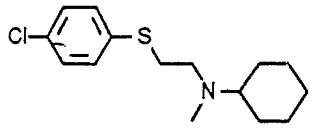 | Refractive Index (26 C) 1.5678 |
| No. 42 | 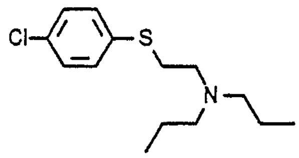 | Refractive Index (26 C) 1.5465 |
| No. 43 | 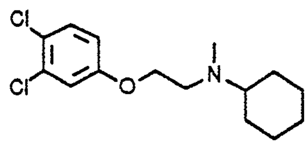 | Refractive Index (26 C) 1.5388 |
| No. 44 | 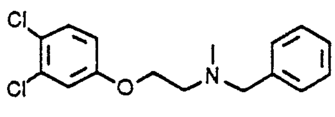 | Refractive Index (26 C) 1.5640 |
| No. 45 | 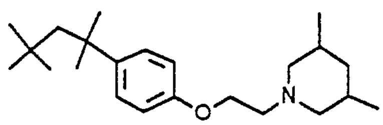 | Refractive Index (26 C) 1.5037 |
| No. 46 | 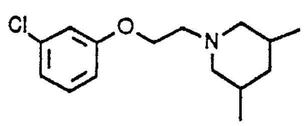 | Refractive Index (26 C) 1.5163 |
| No. 47 | 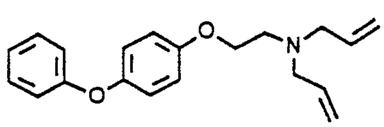 | Refractive Index (26 C) 1.5530 |
| No. 48 | 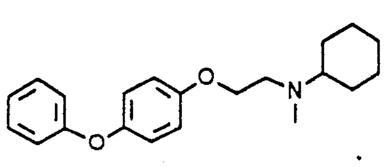 | Refractive Index (26 C) 1.5571 |

No. 49 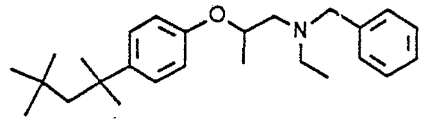 Refractive Index (26 C) 1.5236

No. 50 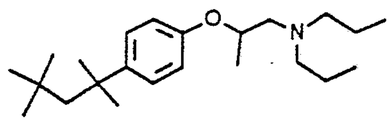 Refractive Index (26 C) 1.4894

No. 51 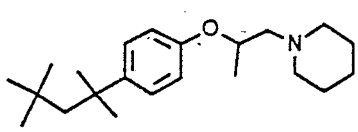 Refractive Index (26 C) 1.5055

No. 52 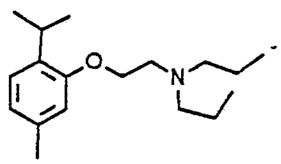 Refractive Index (26 C) 1.4923

No. 53 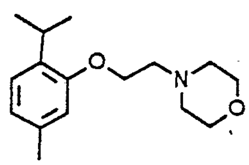 Refractive Index (26 C) 1.5158

No. 54 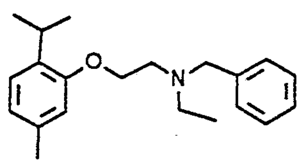 Refractive Index (26 C) 1.5343

No. 55 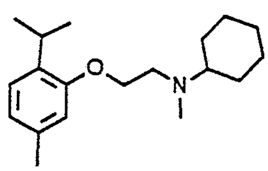 Refractive Index (26 C) 1.5123

No. 56 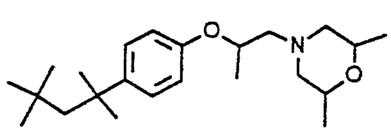 Refractive Index (26 C) 1.4988

| | | |
|---|---|---|
| No. 57 | 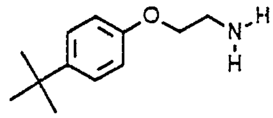 | Refractive Index (26 C) 1.5172 |
| No. 58 | 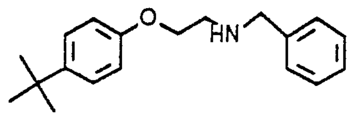 | Refractive Index (26 C) 1.5437 |
| No. 59 | 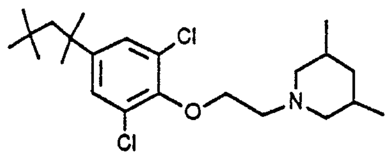 | Refractive Index (26 C) 1.5178 |
| No. 60 | 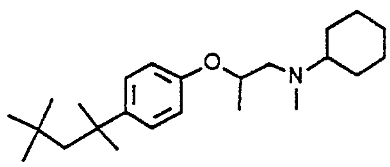 | Refractive Index (26 C) 1.5064 |
| No. 61 | 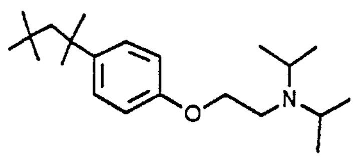 | Refractive Index (26 C) 1.4946 |
| No. 62 | 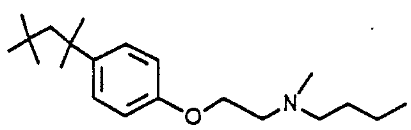 | Refractive Index (26 C) 1.4938 |
| No. 63 | 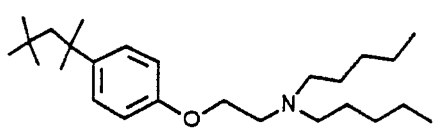 | Refractive Index (26 C) 1.4874 |
| No. 64 | 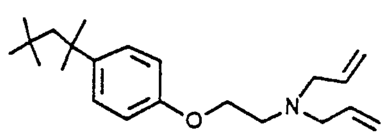 | Refractive Index (26 C) 1.5033 |

| | | |
|---|---|---|
| No. 65 | 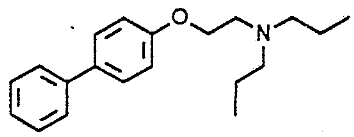 | Refractive Index (26 C) 1.5534 |
| No. 66 | 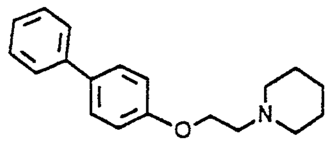 | Solids |
| No. 67 | 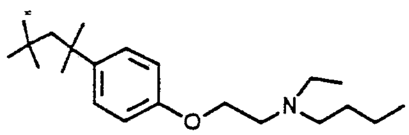 | Refractive Index (26 C) 1.4926 |
| No. 68 | 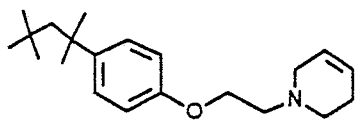 | Refractive Index (26 C) 1.5207 |
| No. 69 | 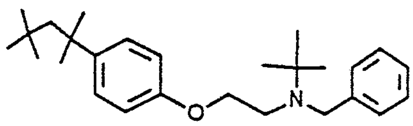 | Refractive Index (26 C) 1.5247 |
| No. 70 | 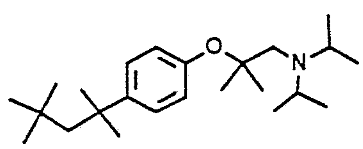 | Refractive Index (26 C) 1.4887 |
| No. 71 | 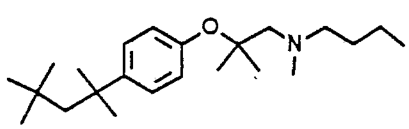 | Refractive Index (26 C) 1.4892 |
| No. 72 | 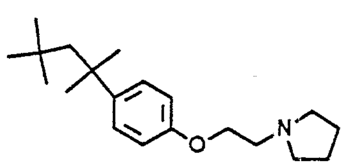 | Refractive Index (26 C) 1.5115 |

| | | |
|---|---|---|
| No. 73 | 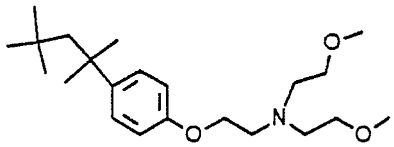 | Refractive Index (26 C) 1.4940 |
| No. 74 | 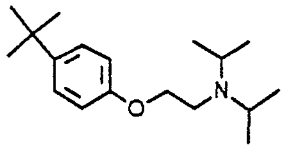 | Refractive Index (26 C) 1.4948 |
| No. 75 | 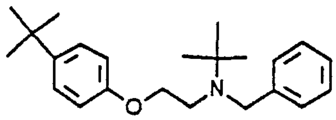 | Refractive Index (26 C) 1.5282 |
| No. 76 | 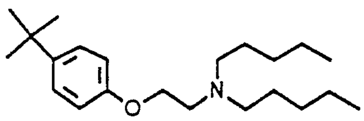 | Refractive Index (26 C) 1.4834 |
| No. 77 | 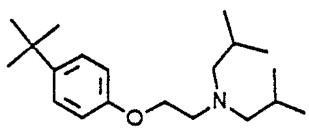 | Refractive Index (26 C) 1.4824 |
| No. 78 | 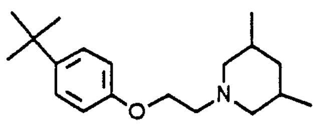 | Refractive Index (26 C) 1.5022 |
| No. 79 | 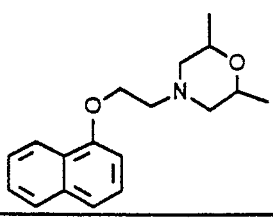 | Refractive Index (26 C) 1.5637 |
| No. 80 | 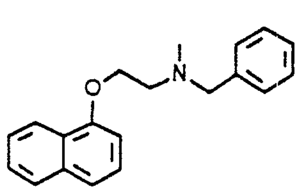 | Refractive Index (26 C) 1.6018 |

| | | |
|---|---|---|
| No. 81 | 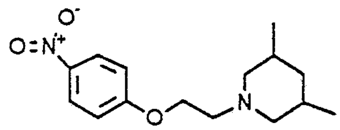 | Refractive Index (26 C) 1.5411 |
| No. 82 | 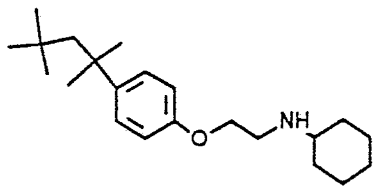 | Refractive Index (26 C) 1.5113 |
| No. 83 | 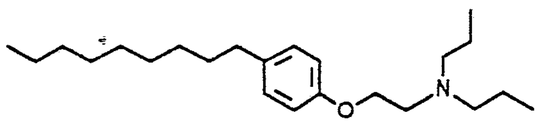 | Refractive Index (26 C) 1.4938 |
| No. 84 | 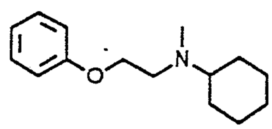 | Refractive Index (26 C) 1.5172 |
| No. 85 | 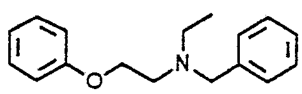 | Refractive Index (26 C) 1.5441 |
| No. 86 | 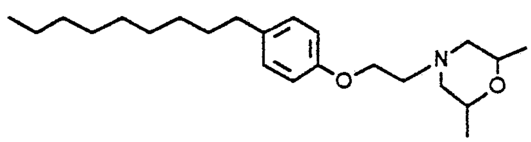 | Refractive Index (26 C) 1.5508 |
| No. 87 | 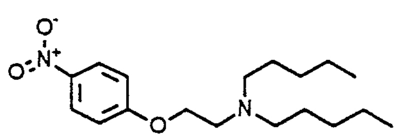 | Refractive Index (26 C) 1.5104 |
| No. 88 | 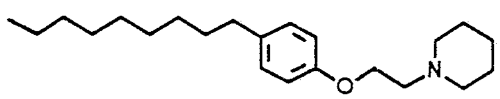 | Refractive Index (26 C) 1.5116 |

| | | |
|---|---|---|
| No. 89 | 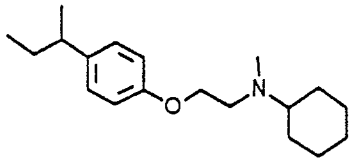 | Refractive Index (26 C) 1.5109 |
| No. 90 | 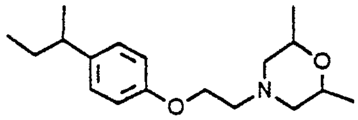 | Refractive Index (26 C) 1.5035 |
| No. 91 | 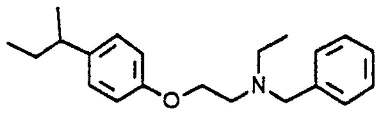 | Refractive Index (26 C) 1.5310 |
| No. 92 | 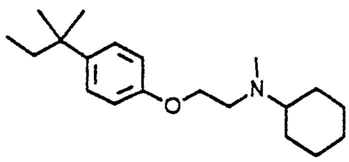 | Refractive Index (26 C) 1.5136 |
| No. 93 | 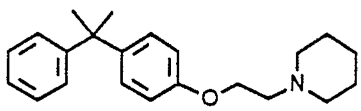 | Refractive Index (26 C) 1.5627 |
| No. 94 | 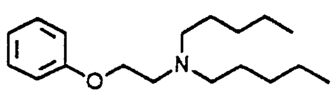 | Refractive Index (26 C) 1.4841 |
| No. 95 | 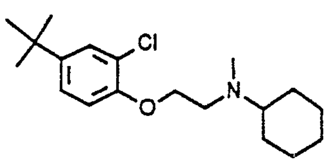 | Refractive Index (26 C) 1.5224 |
| No. 96 | 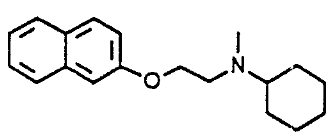 | Refractive Index (26 C) 1.5725 |

| | | |
|---|---|---|
| No. 97 | 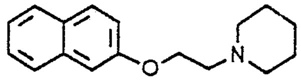 | Refractive Index (26 C) 1.5792 |
| No. 98 | 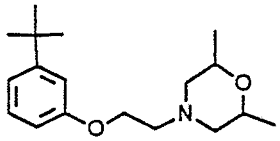 | Refractive Index (26 C) 1.5053 |
| No. 99 | 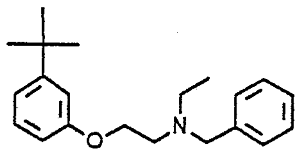 | Refractive Index (26 C) 1.5332 |
| No. 100 | 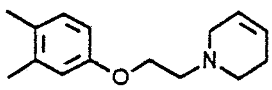 | Refractive Index (26 C) 1.5355 |
| No. 101 | 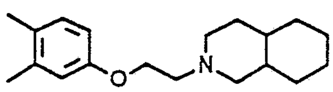 | Refractive Index (26 C) 1.5302 |
| No. 102 | 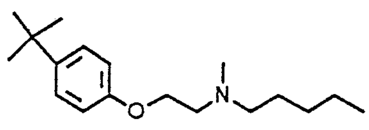 | Refractive Index (26 C) 1.4904 |
| No. 103 | 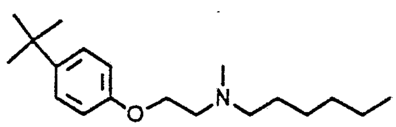 | Refractive Index (26 C) 1.4885 |
| No. 104 | 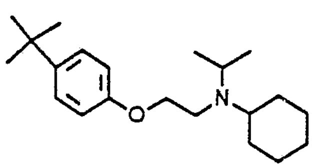 | Refractive Index (26 C) 1.5555 |

No. 105

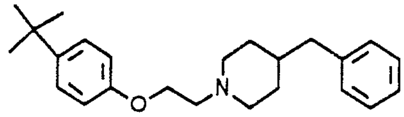

Refractive Index (26 C) 1.5427

---

No. 106

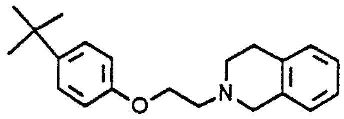

Refractive Index (26 C) 1.5579

---

No. 107

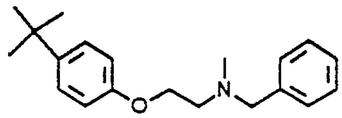

Refractive Index (26 C) 1.5362

---

No. 108

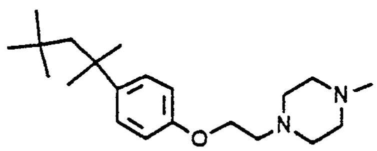

Refractive Index (26 C) 1.5150

---

No. 109

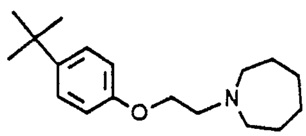

Refractive Index (26 C) 1.5152

---

No. 110

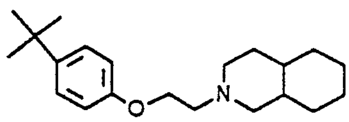

Refractive Index (26 C) 1.5226

---

No. 111

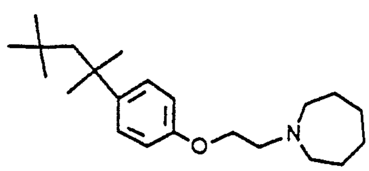

Refractive Index (26 C) 1.5138

---

No. 112

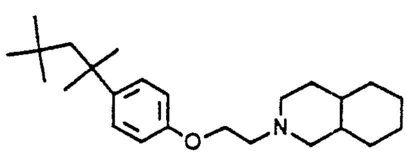

Refractive Index (26 C) 1.5199

---

| | | |
|---|---|---|
| No. 113 | 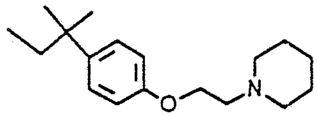 | Refractive Index (26 C) 1.5149 |
| No. 114 | 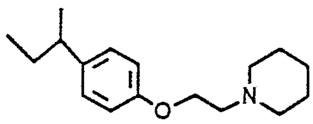 | Refractive Index (26 C) 1.5127 |
| No. 115 | 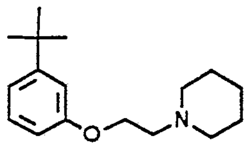 | Refractive Index (26 C) 1.5147 |
| No. 116 | 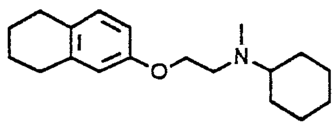 | Refractive Index (26 C) 1.5337 |
| No. 117 | 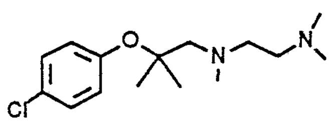 | Refractive Index (26 C) 1.5006 |
| No. 118 | 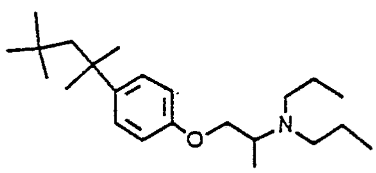 | Refractive Index (26 C) 1.4931 |
| No. 119 | 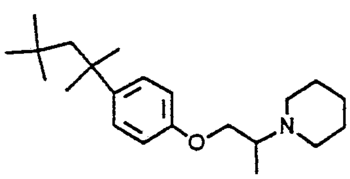 | Refractive Index (26 C) 1.5089 |
| No. 120 | 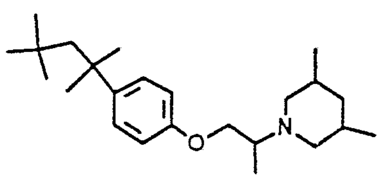 | Refractive Index (26 C) 1.5022 |

| | | |
|---|---|---|
| No. 121 | 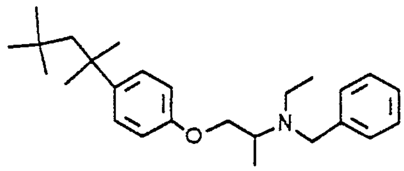 | Refractive Index (26 C) 1.5225 |
| No. 122 | 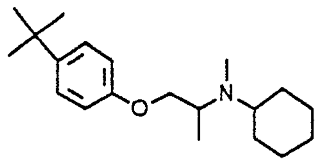 | Refractive Index (26 C) 1.5102 |
| No. 123 | 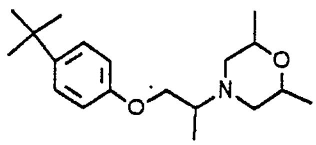 | Refractive Index (26 C) 1.5030 |
| No. 124 | 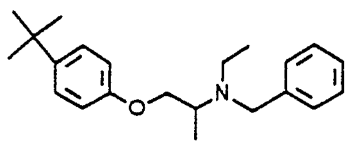 | Refractive Index (26 C) 1.5300 |
| No. 125 | 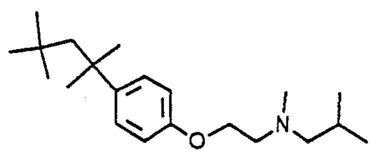 | Refractive Index (26 C) 1.4909 |
| No. 126 | 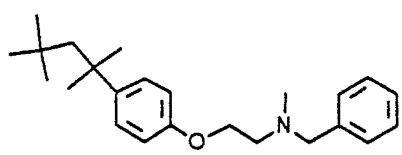 | Refractive Index (26 C) 1.5305 |
| No. 127 | 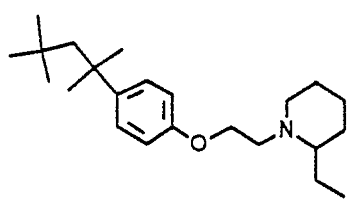 | Refractive Index (26 C) 1.5099 |
| No. 128 | 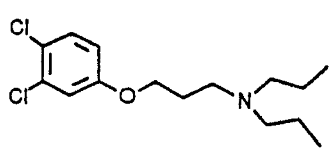 | Refractive Index (26 C) 1.5090 |

| | | |
|---|---|---|
| No. 129 | 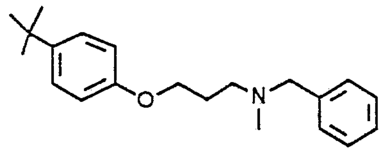 | Refractive Index (26 C) 1.5318 |
| No. 130 | 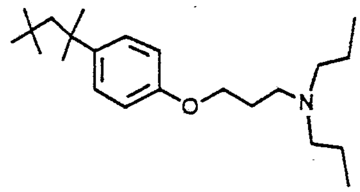 | Refractive Index (26 C) 1.4904 |
| No. 131 | 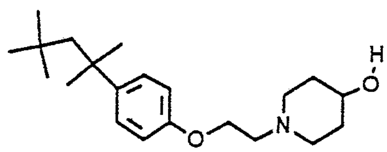 | Viscous |
| No. 132 | 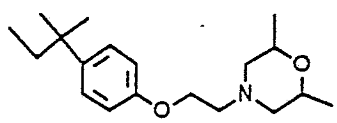 | Refractive Index (26 C) 1.5061 |
| No. 133 | 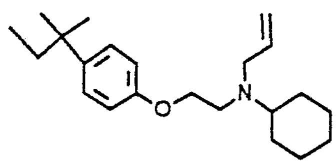 | Refractive Index (26 C) 1.5137 |
| No. 134 | 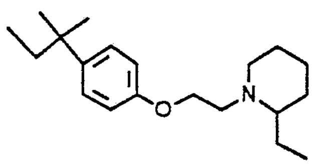 | Refractive Index (26 C) 1.5122 |
| No. 135 | 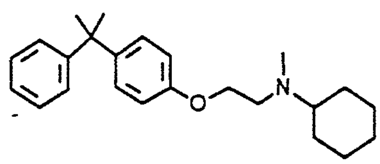 | Refractive Index (26 C) 1.5509 |
| No. 136 | 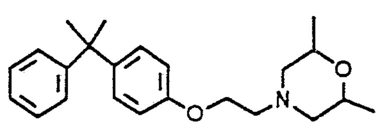 | Refractive Index (26 C) 1.5470 |

| | | |
|---|---|---|
| No. 137 | 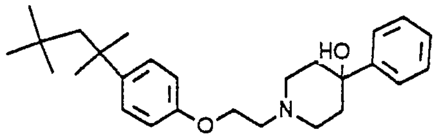 | Viscous |
| No. 138 | 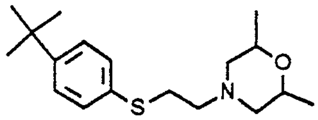 | Refractive Index (26 C) 1.5356 |
| No. 139 | 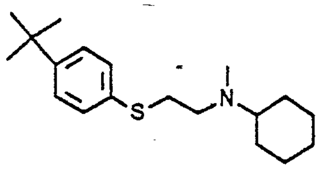 | Refractive Index (26 C) 1.5443 |
| No. 140 | 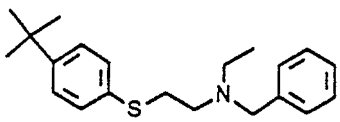 | Refractive Index (26 C) 1.5584 |
| No. 141 | 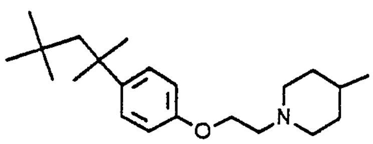 | Refractive Index (26 C) 1.5064 |
| No. 142 | 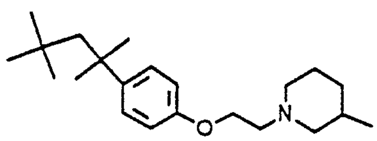 | Refractive Index (26 C) 1.5069 |
| No. 143 | 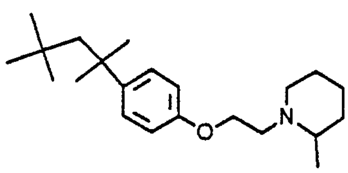 | Refractive Index (26 C) 1.5099 |
| No. 144 | 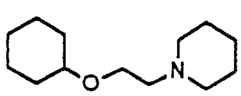 | Refractive Index (26 C) 1.4761 |

No. 145

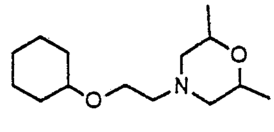

Refractive Index (26 C) 1.4671

---

No. 146

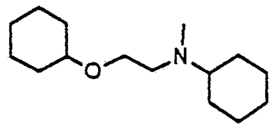

Refractive Index (26 C) 1.4782

---

No. 147

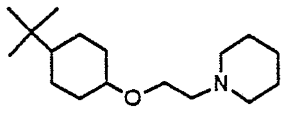

Refractive Index (26 C) 1.4761

---

No. 148

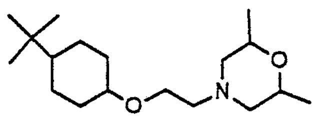

Refractive Index (26 C) 1.4696

---

No. 149

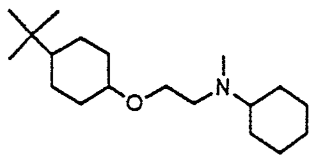

Refractive Index (26 C) 1.4789

---

No. 150

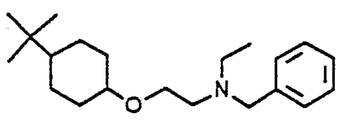

Refractive Index (26 C) 1.5007

---

No. 151

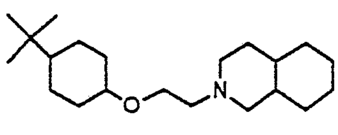

Refractive Index (26 C) 1.4907

---

No. 152

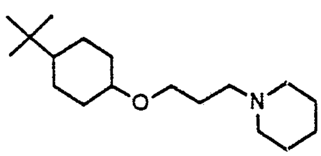

Refractive Index (26 C) 1.4755

---

| | | |
|---|---|---|
| No. 153 | 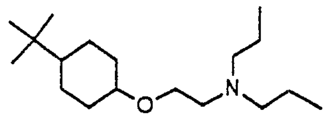 | Refractive Index (26 C) 1.4553 |
| No. 154 | 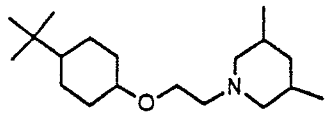 | Refractive Index (26 C) 1.4708 |
| No. 155 | 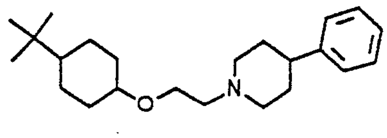 | Refractive Index (26 C) 1.5161 |
| No. 156 | 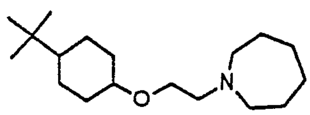 | Refractive Index (26 C) 1.4799 |
| No. 157 | 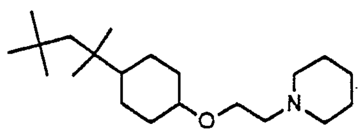 | Refractive Index (26 C) 1.4831 |
| No. 158 | 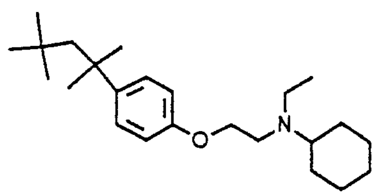 | Refractive Index (26 C) 1.5081 |
| No. 159 | 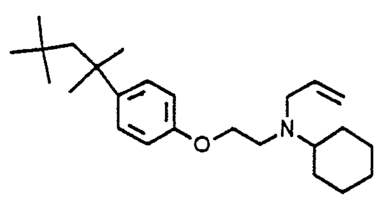 | Refractive Index (26 C) 1.5121 |
| No. 160 | 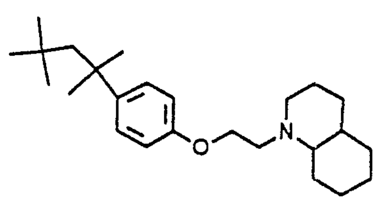 | Refractive Index (26 C) 1.5226 |

| No. | Structure | Refractive Index (26 C) |
|---|---|---|
| No. 161 | 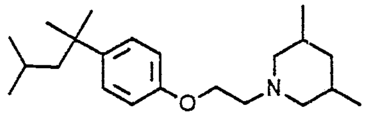 | Refractive Index (26 C) 1,5023 |
| No. 162 | 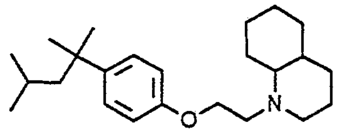 | Refractive Index (26 C) 1.5218 |
| No. 163 | 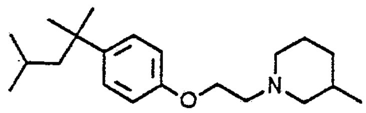 | Refractive Index (26 C) 1.5060 |
| No. 164 | 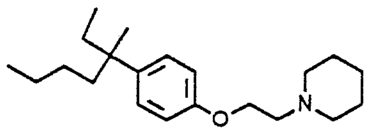 | Refractive Index (26 C) 1.5099 |
| No. 165 | 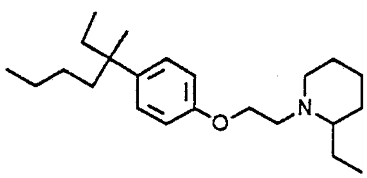 | Refractive Index (26 C) 1.5073 |
| No. 166 | 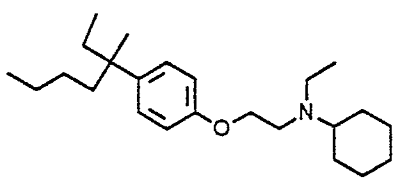 | Refractive Index (26 C) 1.5055 |
| No. 167 | 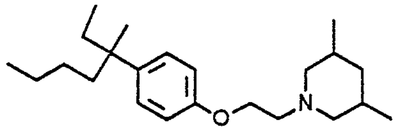 | Refractive Index (26 C) 1.5023 |
| No. 168 | 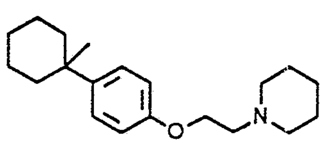 | Refractive Index (26 C) 1.5333 |

No. 169 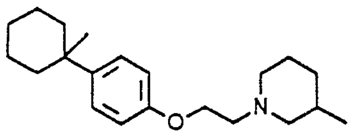 Refractive Index (26 C) 1.5271

No. 170 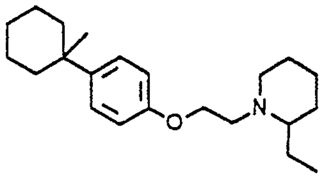 Refractive Index (26 C) 1.5291

No. 171 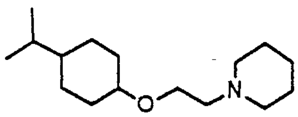 Refractive Index (26 C) 1.4768

No. 172 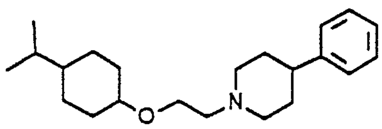 Refractive Index (26 C) 1.5166

No. 173 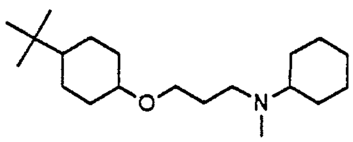 Refractive Index (26 C) 1.4787

No. 174 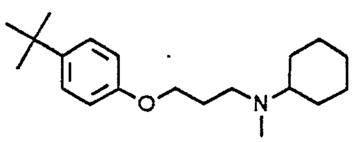 Refractive Index (26 C) 1.5118

No. 175 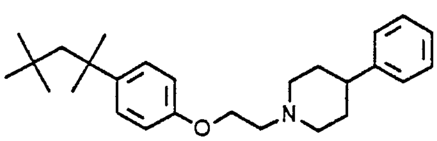 Refractive Index (26 C) 1.5416

No. 176 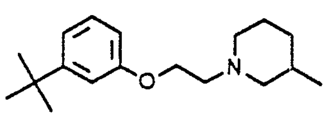 Refractive Index (26 C) 1.5086

| | | |
|---|---|---|
| No. 177 | 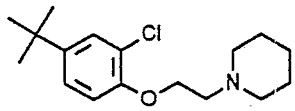 | Refractive Index (26 C) 1.5233 |
| No. 178 | 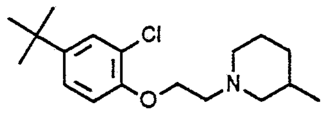 | Refractive Index (26 C) 1.5172 |
| No. 179 | 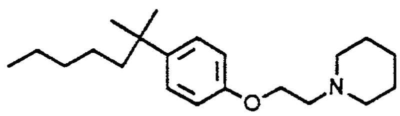 | Refractive Index (26 C) 1.5084 |
| No. 180 | 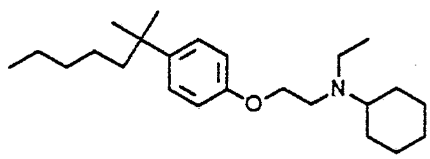 | Refractive Index (26 C) 1.5044 |
| No. 181 | 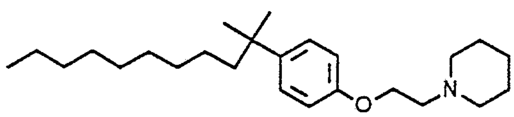 | Refractive Index (26 C) 1.5014 |
| No. 182 | 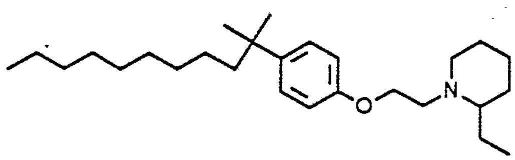 | Refractive Index (26 C) 1.5005 |
| No. 183 | 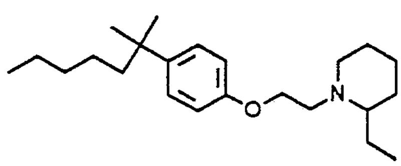 | Refractive Index (26 C) 1.5067 |
| No. 184 | 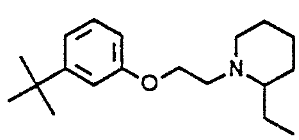 | Refractive Index (26 C) 1.5116 |

| | | |
|---|---|---|
| No. 185 | 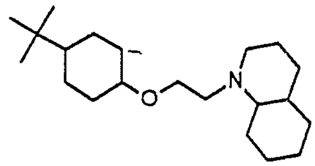 | Refractive Index (26 C) 1.4946 |
| No. 186 | 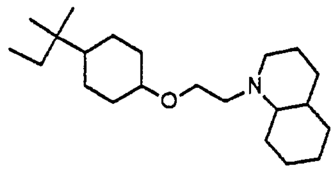 | Refractive Index (26 C) 1.4966 |
| No. 187 | 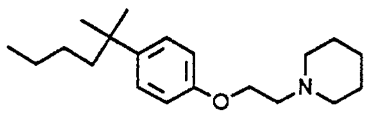 | Refractive Index (26 C) 1.5103 |
| No. 188 | 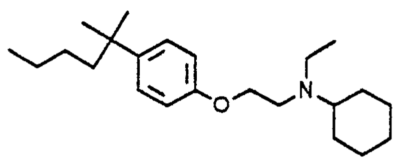 | Refractive Index (26 C) 1.5067 |
| No. 189 | 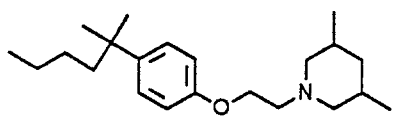 | Refractive Index (26 C) 1.5037 |
| No. 190 | 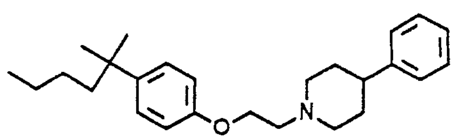 | Refractive Index (26 C) 1.5408 |
| No. 191 | 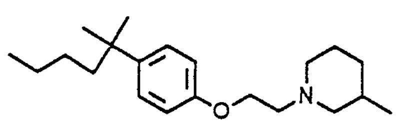 | Refractive Index (26 C) 1.5063 |
| No. 192 | 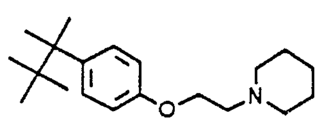 | Refractive Index (26 C) 1.5207 |

No. 193

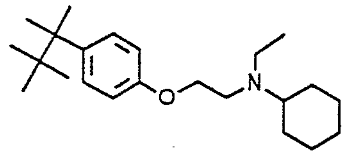

Refractive Index (26 C)
1.5148

---

No. 194

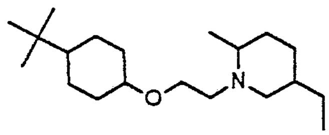

Refractive Index (26 C)
1.4750

---

No. 195

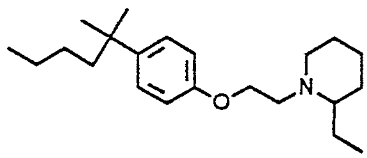

Refractive Index (26 C)
1.5086

---

No. 196

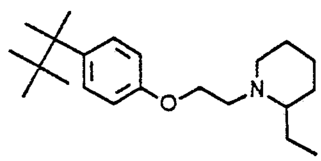

Refractive Index (26 C)
1.5176

---

No. 197

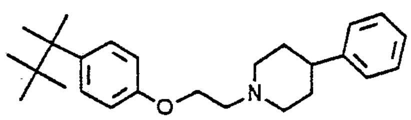

Refractive Index (26 C)
1.5495

---

No. 198

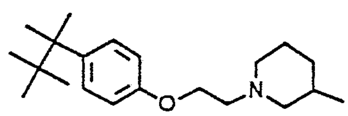

Refractive Index (26 C)
1.5158

---

No. 199

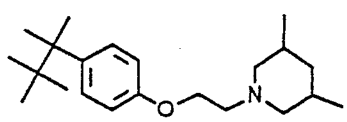

Refractive Index (26 C)
1.5110

---

No. 200

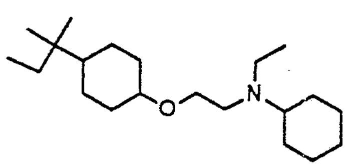

Refractive Index (26 C)
1.4808

---

| | | |
|---|---|---|
| No. 201 | 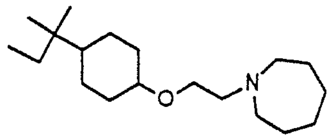 | Refractive Index (26 C) 1.4831 |
| No. 202 | 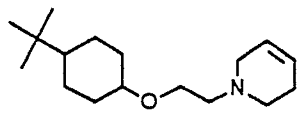 | Refractive Index (26 C) 1.4861 |
| No. 203 | 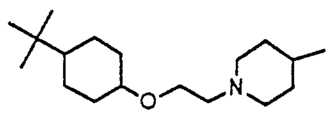 | Refractive Index (26 C) 1.4725 |
| No. 204 | 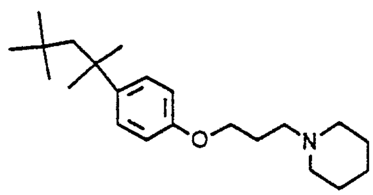 | Refractive Index (26 C) 1.5081 |
| No. 205 | 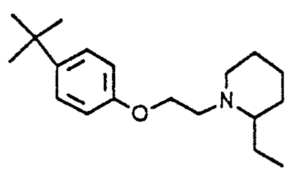 | Refractive Index (26 C) 1.5113 |
| No. 206 | 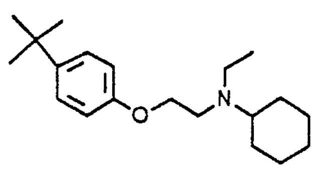 | Refractive Index (26 C) 1.5084 |
| No. 207 | 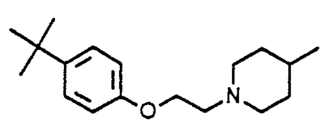 | Melting Point 43 - 48$^{o}$C |
| No. 208 | 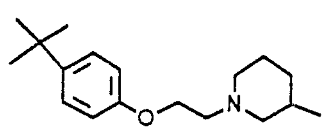 | Refractive Index (26 C) 1.5082 |

| No. | Structure | Refractive Index (26 C) |
|---|---|---|
| 209 | 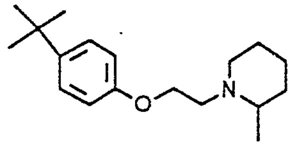 | 1.5111 |
| 210 | 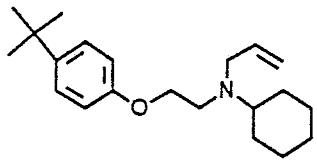 | 1.5136 |
| 211 | 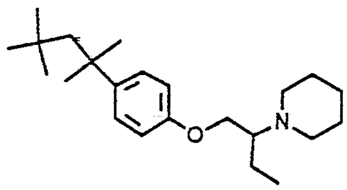 | 1.5072 |
| 212 | 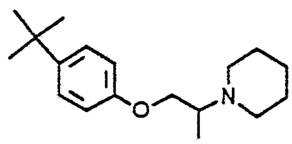 | 1.5112 |
| 213 | 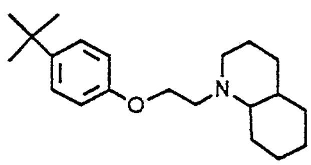 | 1.5253 |
| 214 | 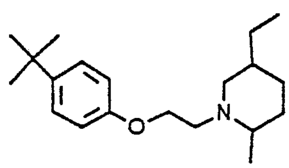 | 1.5047 |
| 215 | 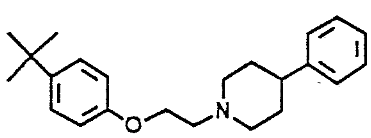 | 1.5478 |
| 216 | 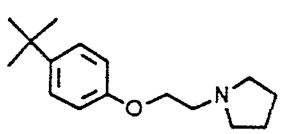 | 1.5146 |

No. 217

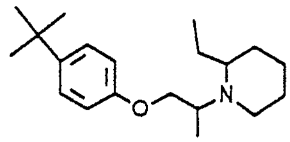

Refractive Index (26 C) 1.5094

---

No. 218

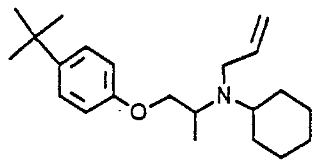

Refractive Index (26 C) 1.5111

---

No. 219

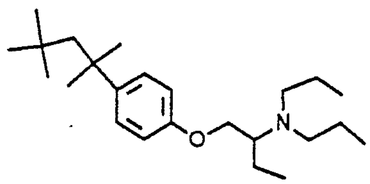

Refractive Index (26 C) 1.4906

---

No. 220

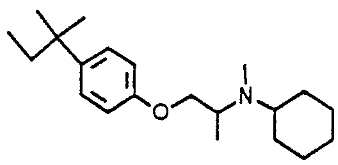

Refractive Index (26 C) 1.5115

---

No. 221

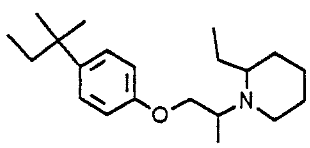

Refractive Index (26 C) 1.5104

---

No. 222

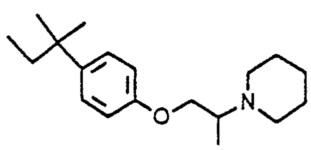

Refractive Index (26 C) 1.5120

---

No. 223

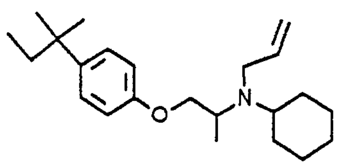

Refractive Index (26 C) 1.5116

---

No. 224

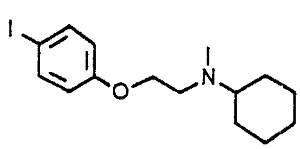

Refractive Index (26 C) 1.5597

---

| | | |
|---|---|---|
| No. 225 | 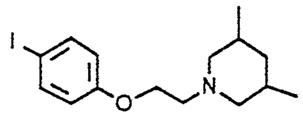 | Refractive Index (26 C) 1.5422 |
| No. 226 | 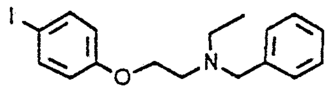 | Refractive Index (26 C) 1.5791 |
| No. 227 | 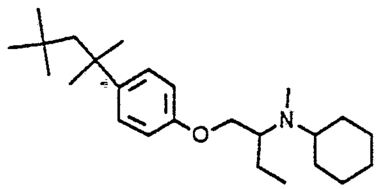 | Refractive Index (26 C) 1.5074 |
| No. 228 | 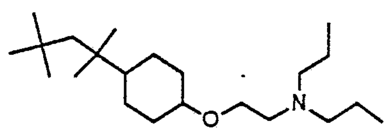 | Refractive Index (26 C) 1.4657 |
| No. 229 | 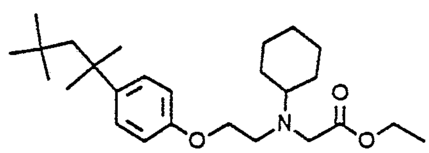 | Refractive Index (26 C) 1.5048 |
| No. 230 | 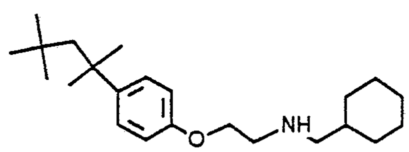 | Refractive Index (26 C) 1.5090 |
| No. 231 | 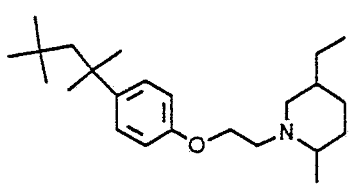 | Refractive Index (26 C) 1.5055 |
| No. 232 | 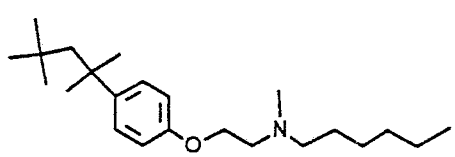 | Refractive Index (26 C) 1.4915 |

No. 233 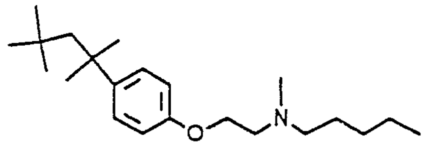 Refractive Index (26 C) 1.4930

No. 234 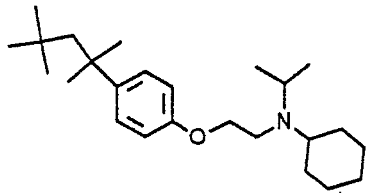 Refractive Index (26 C) 1.5072

No. 235 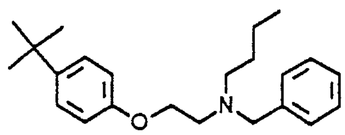 Refractive Index (26 C) 1.5296

No. 236 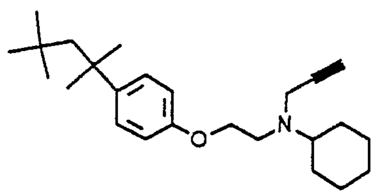 Refractive Index (26 C) 1.5188

No. 237 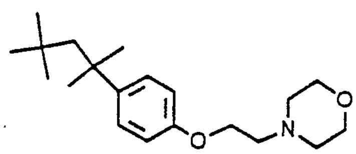 Refractive Index (26 C) 1.5134

No. 238 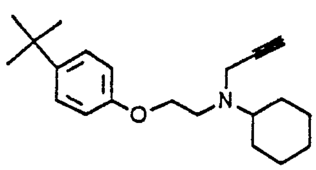 Refractive Index (26 C) 1.5200

No. 239 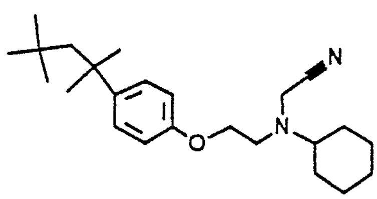 Refractive Index (26 C) 1.5144

No. 240 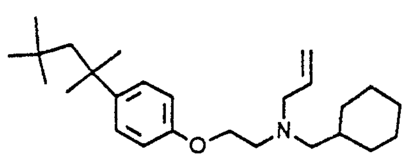 Refractive Index (26 C) 1.5072

| | | |
|---|---|---|
| No. 241 | 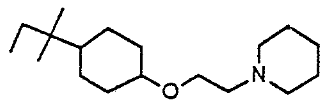 | Refractive Index (26 C) 1.4800 |
| No. 242 | 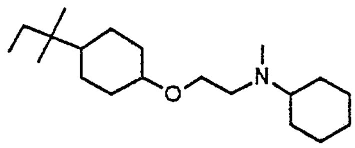 | Refractive Index (26 C) 1.4813 |
| No. 243 | 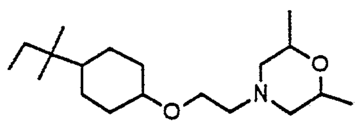 | Refractive Index (26 C) 1.4720 |
| No. 244 | 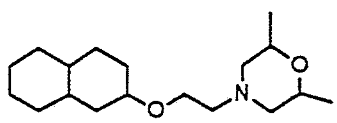 | Refractive Index (26 C) 1.4833 |
| No. 245 | 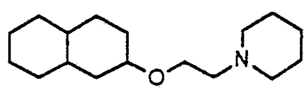 | Refractive Index (26 C) 1.4944 |
| No. 246 | 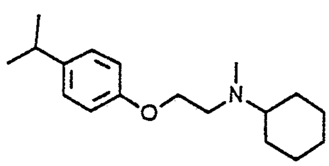 | Refractive Index (26 C) 1.5126 |
| No. 247 | 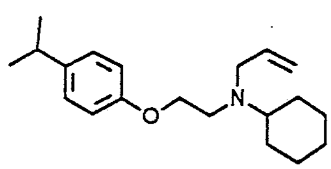 | Refractive Index (26 C) 1.5137 |
| No. 248 | 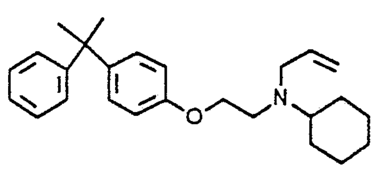 | Refractive Index (26 C) 1.5504 |

No. 249

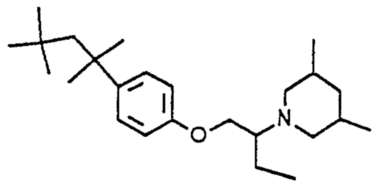

Refractive Index (26 C) 1.5008

---

No. 250

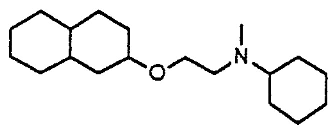

Refractive Index (26 C) 1.4950

---

No. 251

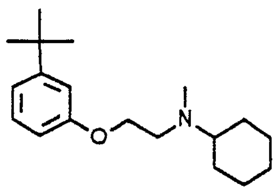

Refractive Index (26 C) 1.5120

---

No. 252

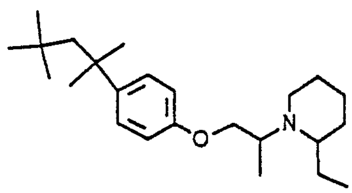

Refractive Index (26 C) 1.5077

---

No. 253

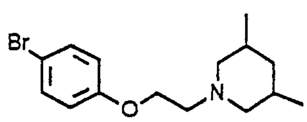

Refractive Index (26 C) 1.5308

---

No. 254

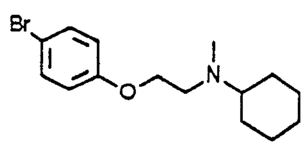

Refractive Index (26 C) 1.5407

---

No. 255

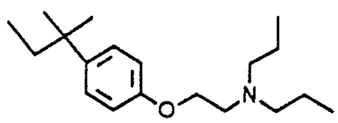

Refractive Index (26 C) 1.4913

---

No. 256

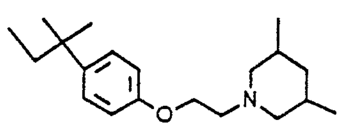

Refractive Index (26 C) 1.5063

---

No. 257

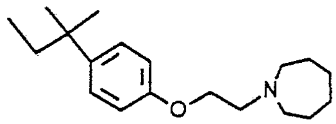

Refractive Index (26 C) 1.5163

---

No. 258

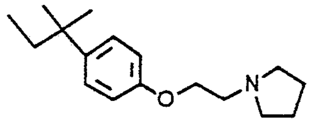

Refractive Index (26 C) 1.5152

---

No. 259

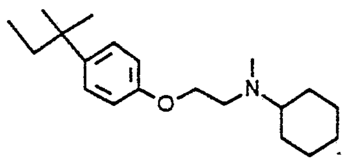

Refractive Index (26 C) 1.5097

---

No. 260

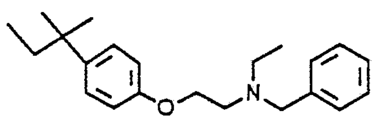

Refractive Index (26 C) 1.5320

---

No. 261

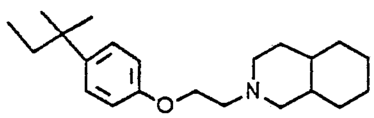

Refractive Index (26 C) 1.5228

---

No. 262

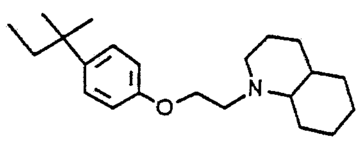

Refractive Index (26 C) 1.5258

---

No. 263

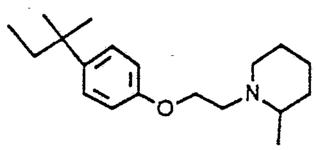

Refractive Index (26 C) 1.5129

---

No. 264

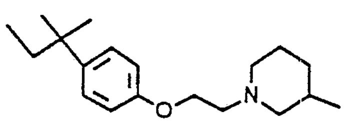

Refractive Index (26 C) 1.5085

---

No. 265 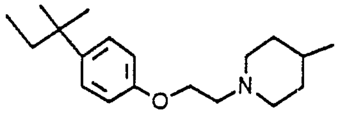 Refractive Index (26 C) 1.5091

No. 266 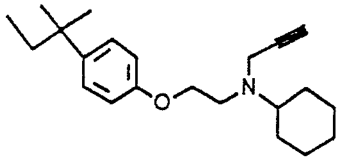 Refractive Index (26 C) 1.5203

No. 267 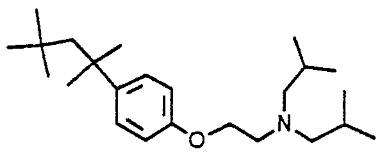 Refractive Index (26 C) 1.4874

No. 268 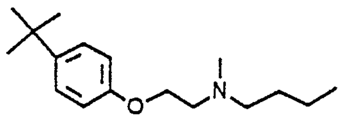 Refractive Index (26 C) 1.4927

No. 269 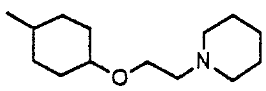 Refractive Index (26 C) 1.4734

No. 270 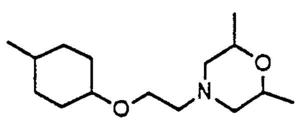 Refractive Index (26 C) 1.4645

No. 271 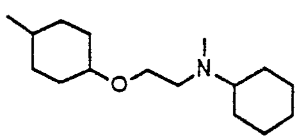 Refractive Index (26 C) 1.4756

No. 272 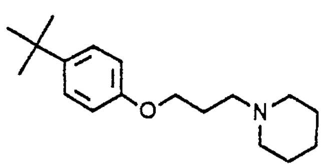 Refractive Index (26 C) 1.5087

No. 273 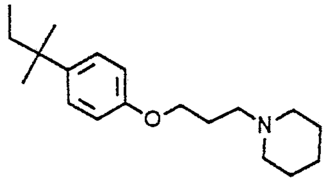 Refractive Index (26 C) 1.5113

No. 274 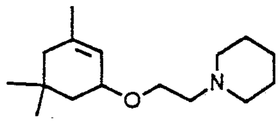 Refractive Index (26 C) 1.4816

No. 275 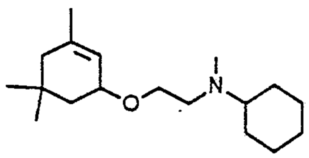 Refractive Index (26 C) 1.4834

No. 276 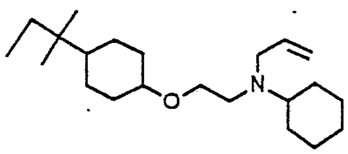 Refractive Index (26 C) 1.4857

No. 277 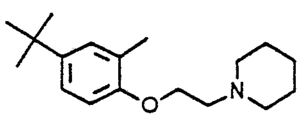 Refractive Index (26 C) 1.5127

No. 278 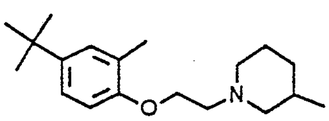 Refractive Index (26 C) 1.5078

No. 279 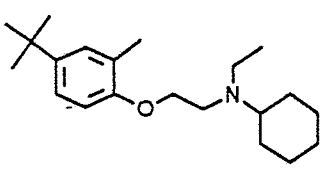 Refractive Index (26 C) 1.5087

No. 280 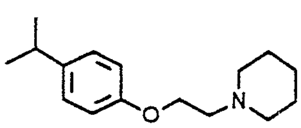 Refractive Index (26 C) 1.5153

No. 281 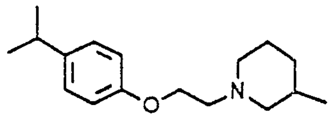 Refractive Index (26 C) 1.5092

---

No. 282 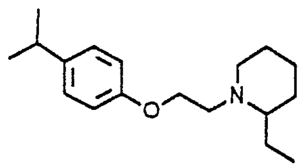 Refractive Index (26 C) 1.5119

---

No. 283 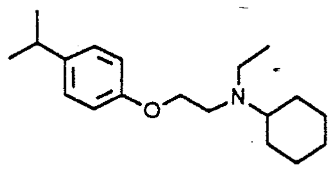 Refractive Index (26 C) 1.5087

---

No. 284 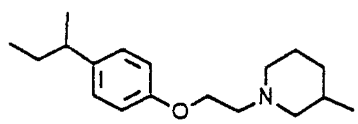 Refractive Index (26 C) 1.5082

---

No. 285 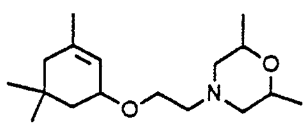 Refractive Index (26 C) 1.4745

---

No. 286 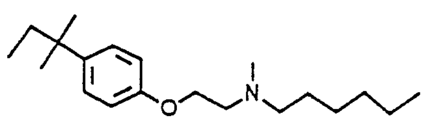 Refractive Index (26 C) 1.4901

---

No. 287 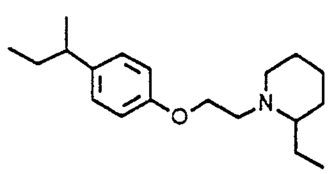 Refractive Index (26 C) 1.5110

---

No. 288 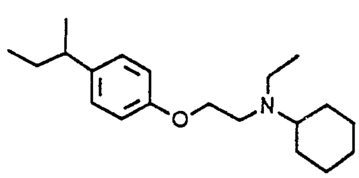 Refractive Index (26 C) 1.5082

---

No. 289 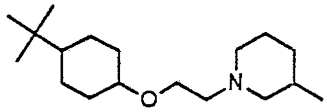 Refractive Index (26 C) 1.4721

No. 290 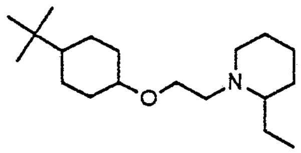 Refractive Index (26 C) 1.4783

No. 291 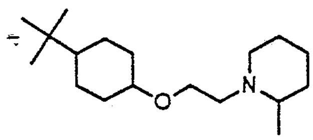 Refractive Index (26 C) 1.4760

No. 292 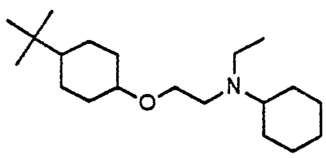 Refractive Index (26 C) 1.4770

No. 293 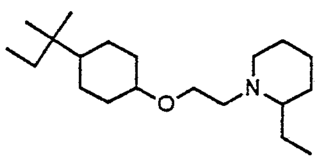 Refractive Index (26 C) 1.4812

No. 294 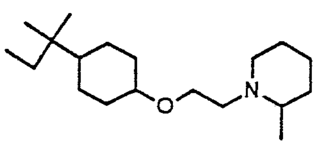 Refractive Index (26 C) 1.4796

No. 295 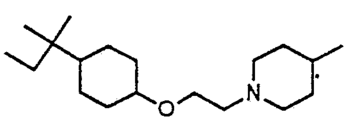 Refractive Index (26 C) 1.4758

No. 296 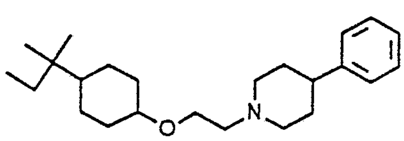 Refractive Index (26 C) 1.5182

No. 297
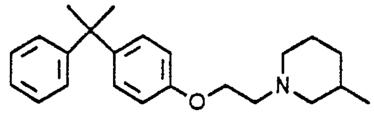
Refractive Index (26 C) 1.5507

No. 298
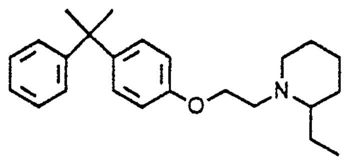
Refractive Index (26 C) 1.5508

No. 299
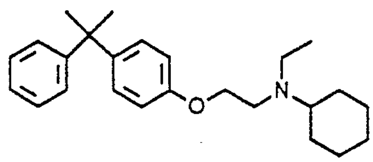
Refractive Index (26 C) 1.5472

No. 300
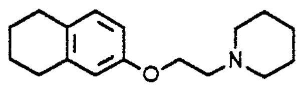
Refractive Index (26 C) 1.5403

No. 301
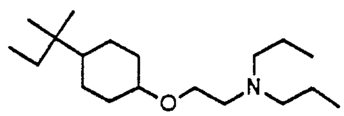
Refractive Index (26 C) 1.4610

No. 302
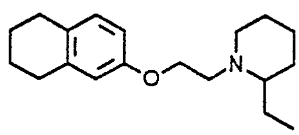
Refractive Index (26 C) 1.5352

No. 303
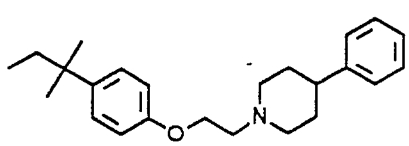
Refractive Index (26 C) 1.5473

No. 304
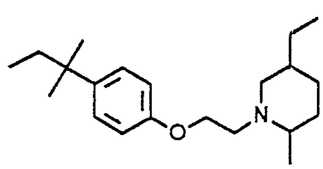
Refractive Index (26 C) 1.5085

| | | |
|---|---|---|
| No. 305 | 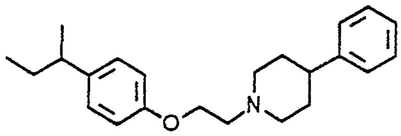 | Refractive Index (26 C) 1.5471 |
| No. 306 | 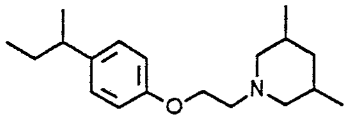 | Refractive Index (26 C) 1.5048 |
| No. 307 | 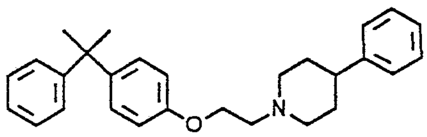 | Refractive Index (26 C) 1.5809 |
| No. 308 | 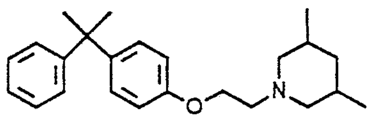 | Refractive Index (26 C) 1.5502 |
| No. 309 | 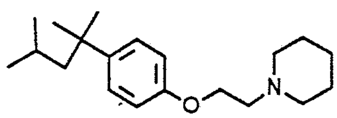 | Refractive Index (26 C) 1.5128 |
| No. 310 | 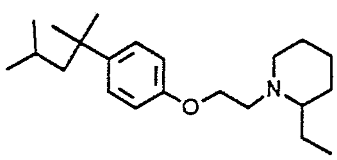 | Refractive Index (26 C) 1.5102 |
| No. 311 | 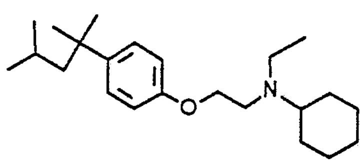 | Refractive Index (26 C) 1.5068 |
| No. 312 | 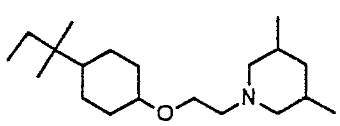 | Refractive Index (26 C) 1.4743 |

| | | |
|---|---|---|
| No. 313 | 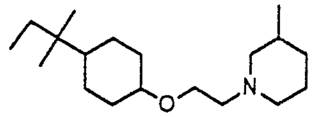 | Refractive Index (26 C) 1.4764 |
| No. 314 | 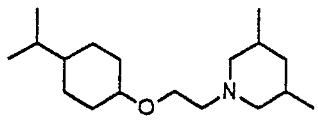 | Refractive Index (26 C) 1.4698 |
| No. 315 | 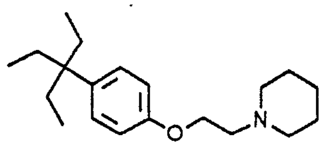 | Refractive Index (26 C) 1.5182 |
| No. 316 | 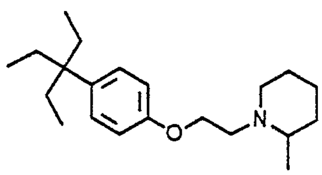 | Refractive Index (26 C) 1.5153 |
| No. 317 | 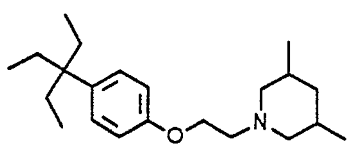 | Refractive Index (26 C) 1.5087 |
| No. 318 | 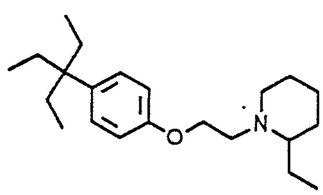 | Refractive Index (26 C) 1.5159 |
| No. 319 | 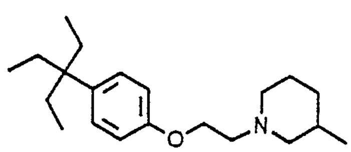 | Refractive Index (26 C) 1.5119 |
| No. 320 | 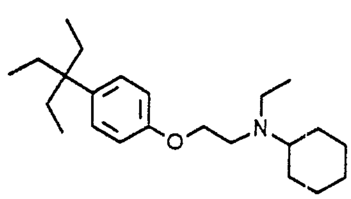 | Refractive Index (26 C) 1.5120 |

| | | |
|---|---|---|
| No. 321 | 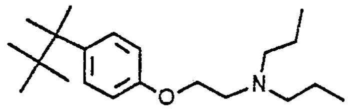 | Refractive Index (26 C) 1.4971 |
| No. 322 | 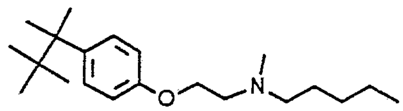 | Refractive Index (26 C) 1.4986 |
| No. 323 | 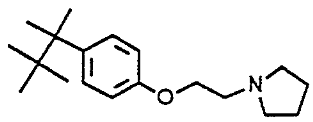 | Refractive Index (26 C) 1.5206 |
| No. 324 | 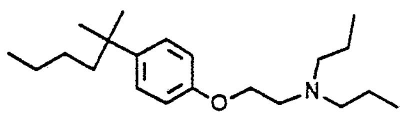 | Refractive Index (26 C) 1.4903 |
| No. 325 | 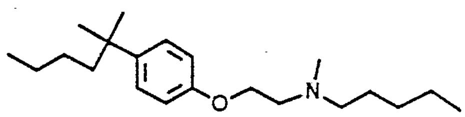 | Refractive Index (26 C) 1.4903 |
| No. 326 | 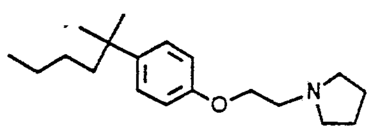 | Refractive Index (26 C) 1.5103 |
| No. 327 | 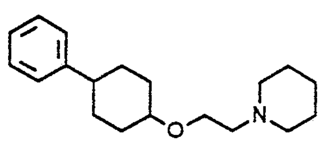 | Refractive Index (26 C) 1.5183 |
| No. 328 | 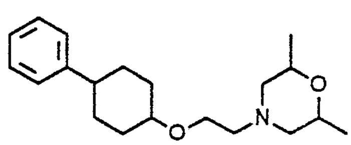 | Refractive Index (26 C) 1.5103 |

| | | |
|---|---|---|
| No. 329 | 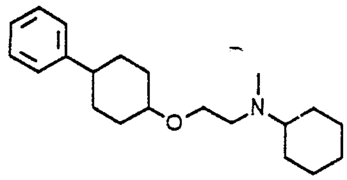 | Refractive Index (26 C) 1.5145 |
| No. 330 | 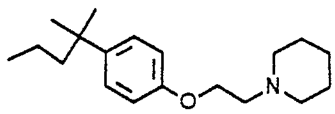 | Refractive Index (26 C) 1.5116 |
| No. 331 | 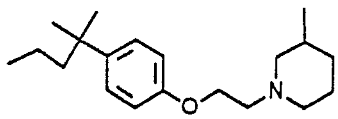 | Refractive Index (26 C) 1.5073 |
| No. 332 | 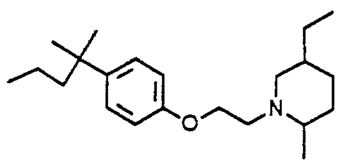 | Refractive Index (26 C) 1.5054 |
| No. 333 | 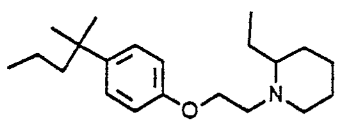 | Refractive Index (26 C) 1.5097 |
| No. 334 | 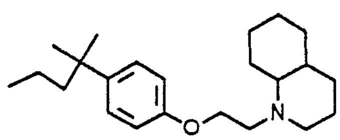 | Refractive Index (26 C) 1.5235 |
| No. 335 | 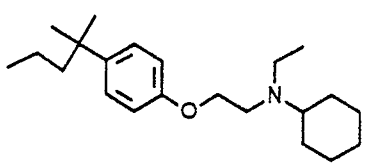 | Refractive Index (26 C) 1.5074 |
| No. 336 | 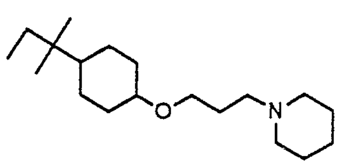 | Refractive Index (26 C) 1.4778 |

| | | |
|---|---|---|
| No. 337 | 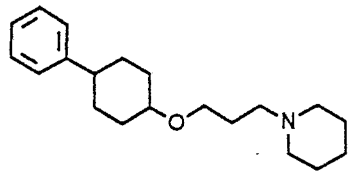 | Refractive Index (26 C) 1.5178 |
| No. 338 | 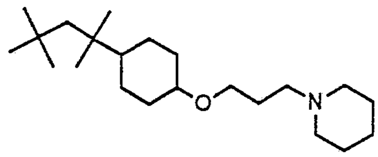 | Refractive Index (26 C) 1.4809 |
| No. 339 | 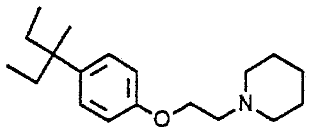 | Refractive Index (26 C) 1.5147 |
| No. 340 | 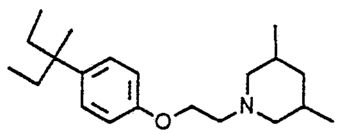 | Refractive Index (26 C) 1.5076 |
| No. 341 | 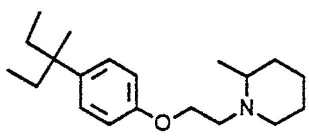 | Refractive Index (26 C) 1.5120 |
| No. 342 | 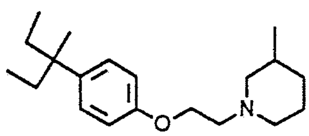 | Refractive Index (26 C) 1.5096 |
| No. 343 | 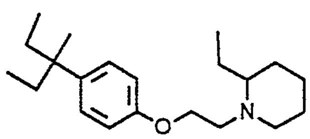 | Refractive Index (26 C) 1.5123 |
| No. 344 | 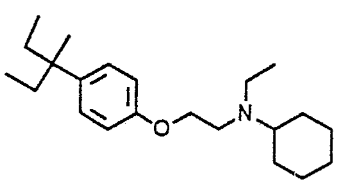 | Refractive Index (26 C) 1.5104 |

| No. | | |
|---|---|---|
| No. 345 | 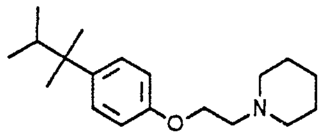 | Refractive Index (26 C) 1.5163 |
| No. 346 | 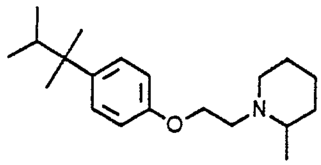 | Refractive Index (26 C) 1.5126 |
| No. 347 | 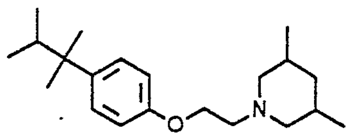 | Refractive Index (26 C) 1.5065 |
| No. 348 | 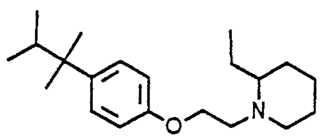 | Refractive Index (26 C) 1.5133 |
| No. 349 | 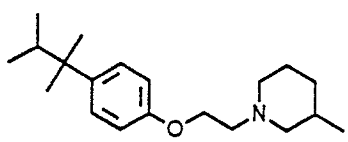 | Refractive Index (26 C) 1.5105 |
| No. 350 | 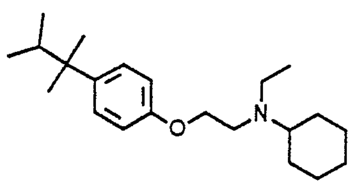 | Refractive Index (26 C) 1.5106 |
| No. 351 | 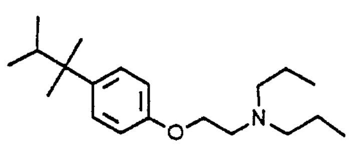 | Refractive Index (26 C) 1.4939 |
| No. 352 | 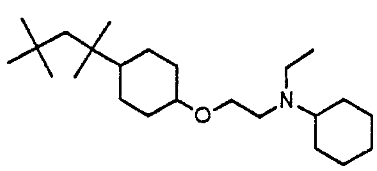 | Refractive Index (26 C) 1.4821 |

| No. | Structure | Refractive Index (26 C) |
|---|---|---|
| 353 | 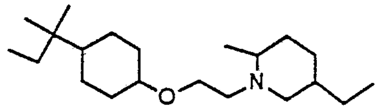 | 1.4773 |
| 354 | 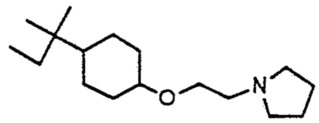 | 1.4773 |
| 355 | 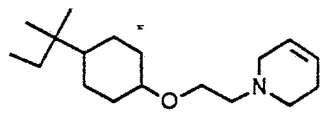 | 1.4883 |
| 356 | 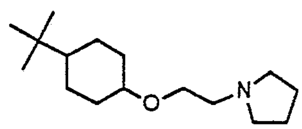 | 1.4742 |
| 357 | 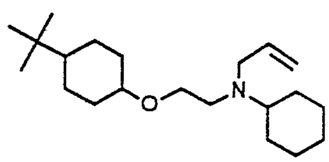 | 1.4821 |
| 358 | 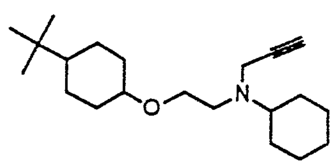 | 1.4840 |
| 359 | 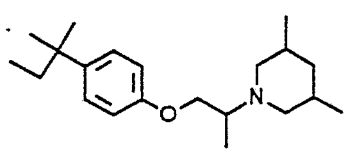 | 1.5035 |
| 360 | 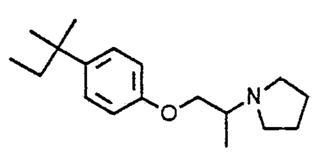 | 1.5123 |

| No. | | Refractive Index (26 C) |
|---|---|---|
| 361 | 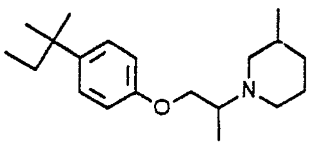 | 1.5073 |
| 362 | 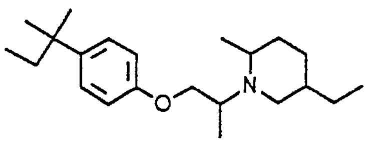 | 1.5056 |
| 363 | 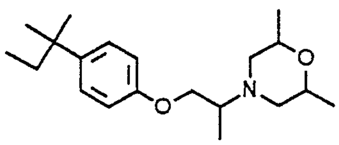 | 1.5042 |
| 364 | 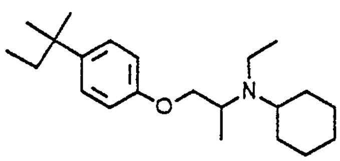 | 1.5075 |
| 365 | 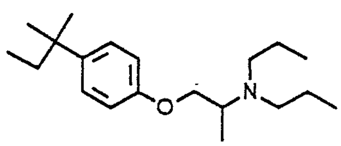 | 1.4905 |

| No. | | ppm[x)] | Substituent |
|---|---|---|---|
| 1001 | 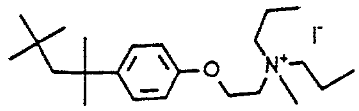 | 49.87 | $CH_3$ |
| 1002 | 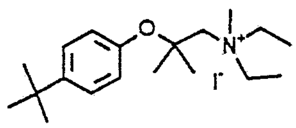 | 49.79 | $CH_3$ |
| 1003 | 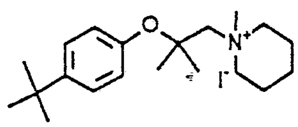 | 49.64 | $CH_3$ |
| 1004 | 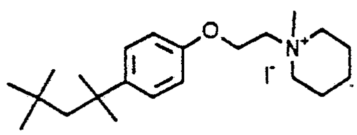 | 49.65 | $CH_3$ |
| 1005 | 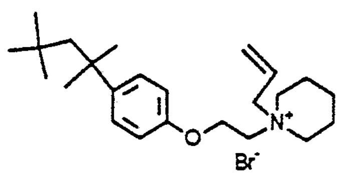 | 61.84 | $CH_2$=CH-$CH_2$- |
| 1006 | 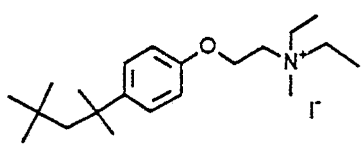 | 48.93 | $CH_3$ |
| 1007 | 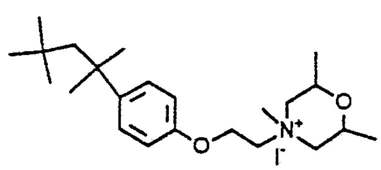 | 47.77 | $CH_3$ |
| 1008 | 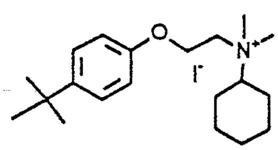 | 49.54 | $CH_3$ |

x) $^{13}C$ chemical shift of the α-C of the substituent.

| No. | | ppm x) | Substituent |
|---|---|---|---|
| 1009 | 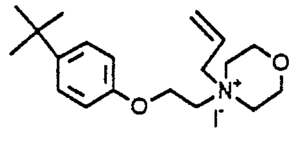 | 62.06 | $CH_2$=CH-$CH_2$- |
| 1010 | 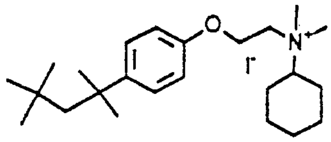 | 49.81 | $CH_3$ |
| 1011 | 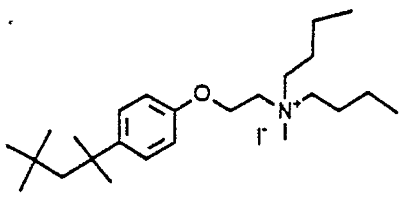 | 49.83 | $CH_3$ |
| 1012 | 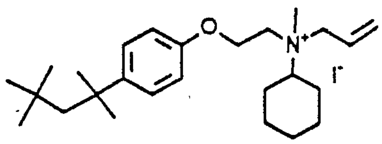 | 62.40 | $CH_2$=CH-$CH_2$- |
| 1013 | 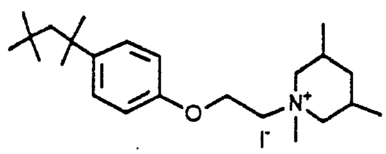 | 47.47 | $CH_3$ |
| 1014 | 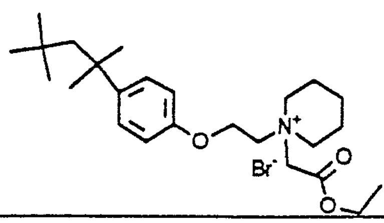 | 61.80 | -$CH_2$-$COOC_2H_5$ |
| 1015 | 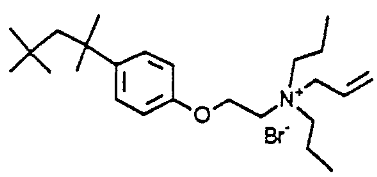 | 62.69 | $CH_2$=CH-$CH_2$- |
| 1016 | 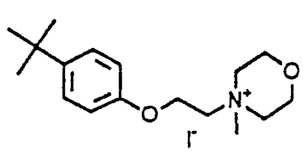 | 49.763 | $CH_3$ |

x) $^{13}C$ chemical shift of the α-C of the substituent.

| No. | | x) ppm | Substituent |
|---|---|---|---|
| 1017 | 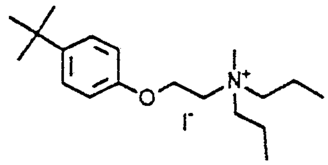 | 49.908 | $CH_3$ |
| 1018 | 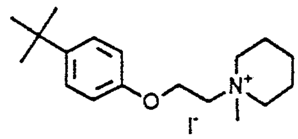 | 49.989 | $CH_3$ |
| 1019 | 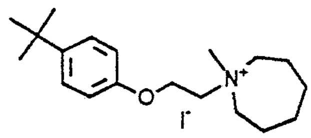 | 52.823 | $CH_3$ |
| 1020 | 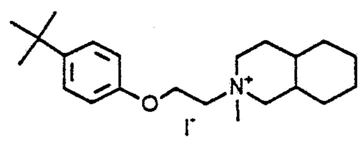 | 47.017 | $CH_3$ |
| 1021 | 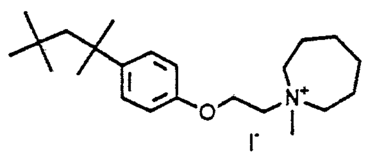 | 52.794 | $CH_3$ |
| 1022 | 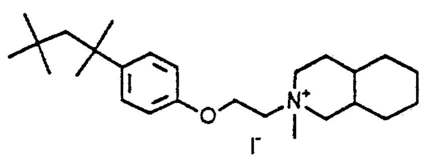 | 47.082 | $CH_3$ |
| 1023 | 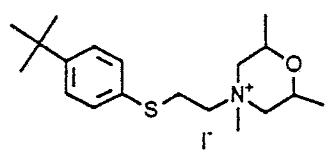 | 46.767 | $CH_3$ |
| 1024 | 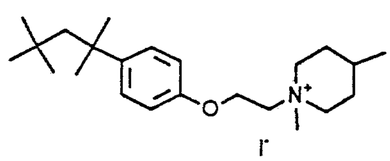 | 46.291 | $CH_3$ |

x) $^{13}C$ chemical shift of the α-C of the substituent.

| No. | | x) ppm | Substituent |
|---|---|---|---|
| 1025 | 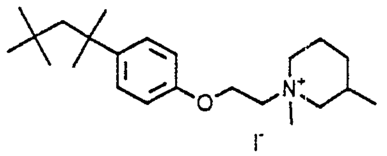 | 46.750 | $CH_3$ |
| 1026 | 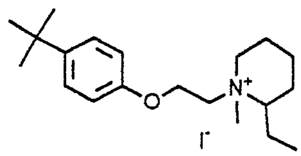 | 43.793 | $CH_3$ |
| 1027 | 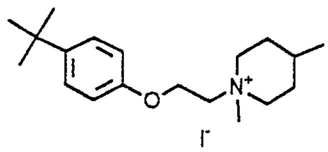 | 46.294 | $CH_3$ |
| 1028 | 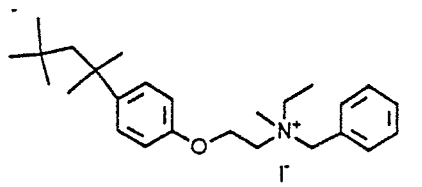 | 47.860 | $CH_3$ |

x) $^{13}C$ chemical shift of the α-C of the substituent.

The compounds of the invention may be formulated as compositions such as wettable powders, dusts, granules, solutions, emulsifiable concentrates, emulsions, suspension concentrates or aerosols.

Wettable powders are usually formulated to contain 25, 50 or 75% of toxicant and usually contain, in addition to solid carrier, 3-10% by weight of a dispersing agent and, where necessary 0-10% by weight of stabilisers and/or additives such as penetrants or stickers.

Dusts are usually formulated as a dust concentrate having a similar composition to that of a wettable powder but without a disperssant and are diluted in the field with further solid carrier to give a composition usually containing 1/2-10% by weight of toxicant. Granules are usually prepared to have a size between 10 and 100 BS mesh, (1.676-0.152 mm) and may be manufactured by agglomeration or impregnation techniques.

Generally, granules will contain 1/2-25% by weight toxicant and 0-10% by weight of additives such as stabilisers, slow release modifiers and binding agents. Emulsifiable concentrates usually contain, in addition to the solvent and, when necessary, co-solvent, 10-50% w/v toxicant, 2-20% w/v emulsifiers and 0-20% w/v of appropriate additives such as stabilisers, penetrants and corrosion inhibitors. Suspension concentrates are compounded so as to obtain a stable, non-sedimenting, flowable product and usually contain 10-75% by weight of toxicant, 1/2-15% by weight of dispersing agents, 0-10% by weight of suspending agents such as protective colloids and thixotropic agents, 0-10% by weight of appropriate additives such as defoamers, corrosion inhibitors, stabilisers, penetrants and stickers, and as carrier, water or an organic liquid in which the toxicant is substantially insoluble. Certain organic solids or inorganic salts may be dissolved in the carrier to assist in preventing sedimentation or as antifreeze agents for water.

Aqueous dispersions and emulsions, for example, compositions obtained by diluting a wettable powder or a concentrate according to the invention with water, also lie within the scope of the present invention. The said emulsions may be of the water-in-oil or of the olie-in-water type, and may have a thick "mayonnaise"-like consistency.

The compositions may also contain other ingredients, for example, other compounds having pesticidal, particularly acaricidal, herbicidal or fungicidal properties.

The activity of the compounds of the invention is illustrated in detail by the following Application Examples.

Application Examples

Eksempel 1

*Erysiphe graminis* test (barley/curative spray).
Solvent : 10% acetone in water.
Emulsifier: Triton X-155, 100 ppm.

For preparing an appropriate composition of active substance the active substance is dissolved in the specified solvent with emulsifier to the concentration desired. Further dilution is carried out by a solution containing 10% acetone in water and 100 ppm Triton X-155.

To test for curative activity young barley plants are sprayed to leaf wetness with the composition of active substance 2 days after the plants have been inoculated with mildew conidia by the brush method. After inoculation and until spraying the plants are left at 18°C and 100° RH, and after spraying they are left in a growth chamber at 18°C and 70-80% RH.

7 Days after inoculation the activity of the substane is evaluated. In this test a clear superiority in activity is shown by, e.g., the compounds listed in Table 1. The activity of the compounds is indicated as given below:

3 90-100% disease control
2 50-89% disease control
1 11-49% disease control
0 0-10% disease control

## Table 1

Erysiphe graminis test

64 ppm curative spray.

| No. | Activity | No. | Activity |
|---|---|---|---|
| 1 | 3 | 137 | 3 |
| 4 | 2 | 141 | 2 |
| 11 | 2 | 142 | 2 |
| 18 | 2 | 143 | 3 |
| 19 | 3 | 147 | 3 |
| 21 | 3 | 148 | 3 |
| 22 | 2 | 149 | 2 |
| 24 | 2 | 150 | 2 |
| 26 | 3 | 151 | 3 |
| 33 | 2 | 152 | 3 |
| 45 | 2 | 153 | 3 |
| 48 | 2 | 154 | 3 |
| 56 | 3 | 155 | 3 |
| 63 | 2 | 156 | 3 |
| 71 | 2 | 157 | 3 |
| 78 | 3 | 158 | 3 |
| 82 | 2 | 159 | 3 |
| 92 | 2 | 160 | 3 |
| 98 | 2 | 204 | 3 |
| 102 | 2 | 205 | 3 |
| 110 | 3 | 206 | 3 |
| 113 | 2 | 207 | 3 |
| 118 | 3 | 208 | 3 |
| 119 | 3 | 209 | 3 |
| 123 | 3 | 210 | 3 |
| 130 | 2 | 212 | 2 |
| 132 | 3 | 213 | 3 |
| 133 | 3 | 214 | 3 |
| 134 | 3 | 215 | 3 |
| 135 | 3 | 217 | 3 |
| 136 | 3 | 220 | 3 |

Table 1 ,continued

*Erysiphe graminis* test

64 ppm curative spray.

| No. | Activity | No. | Activity |
|---|---|---|---|
| 221 | 3 | 290 | 3 |
| 222 | 3 | 291 | 3 |
| 223 | 3 | 292 | 3 |
| 227 | 3 | 293 | 3 |
| 228 | 3 | 294 | 3 |
| 232 | 2 | 295 | 3 |
| 233 | 3 | 296 | 3 |
| 234 | 3 | 297 | 3 |
| 241 | 3 | 301 | 3 |
| 242 | 2 | 303 | 3 |
| 243 | 3 | 305 | 3 |
| 248 | 3 | 306 | 3 |
| 255 | 2 | 308 | 3 |
| 256 | 3 | 312 | 3 |
| 257 | 2 | 313 | 3 |
| 258 | 3 | 314 | 3 |
| 259 | 3 | 1001 | 2 |
| 261 | 2 | 1002 | 2 |
| 262 | 3 | 1003 | 3 |
| 263 | 2 | 1004 | 3 |
| 264 | 3 | 1005 | 2 |
| 265 | 2 | 1006 | 2 |
| 266 | 2 | 1007 | 3 |
| 272 | 3 | 1008 | 2 |
| 273 | 3 | 1009 | 3 |
| 278 | 3 | 1010 | 2 |
| 284 | 3 | 1011 | 2 |
| 286 | 3 | 1012 | 2 |
| 287 | 2 | 1013 | 3 |
| 288 | 3 | 1018 | 3 |
| 289 | 3 | 1019 | 3 |
| | | 1020 | 3 |

Example 2

Puccinia reconditatest (wheat, protective spray).
Solvent : 10% acetone in water.
Emulsifier : Triton X-155, 100 ppm.

For preparing an appropriate composition of active substance the active substance is dissolved in the specified solvent with emulsifier to the concentration desired. Further dilution is carried out by a solution containing 10% acetone in water and 100 ppm Triton X-155.

To test for protective activity young wheat plants are sprayed to leaf wetness with the composition of active substance. After drying the plants are inoculated with brown rust spores in an aqueous slurry which is sprayed on. The plants are left for 48 hours at 18°C and 100% RH, whereafter they are transferred to a growth chamber at the same temperature and 70-80% RH.

10 Days after inoculation the activity of the substance is evaluated. In this test a clear superiority in activity is shown by, e.g., the compounds listed in Tabel 2.

The activity of the compounds is indicated as given below:
3 90-100% disease control
2 50-89% disease control
1 11-49% disease control
0 0-10% disease control

Table 2

Puccinia recondita test
256 ppm protective spray.

| No. | Activity | No. | Activity |
|---|---|---|---|
| 1 | 3 | 109 | 3 |
| 4 | 2 | 110 | 2 |
| 15 | 2 | 113 | 2 |
| 16 | 2 | 118 | 2 |
| 17 | 2 | 126 | 3 |
| 18 | 2 | 127 | 2 |
| 19 | 2 | 129 | 2 |
| 20 | 2 | 130 | 3 |
| 24 | 2 | 133 | 2 |
| 25 | 2 | 134 | 3 |
| 45 | 3 | 135 | 2 |
| 51 | 2 | 136 | 2 |
| 54 | 2 | 142 | 3 |
| 55 | 2 | 143 | 2 |
| 56 | 2 | 147 | 2 |
| 60 | 2 | 148 | 2 |
| 61 | 3 | 149 | 3 |
| 62 | 3 | 151 | 3 |
| 63 | 2 | 152 | 2 |
| 64 | 2 | 153 | 2 |
| 65 | 2 | 154 | 3 |
| 66 | 2 | 155 | 2 |
| 67 | 2 | 156 | 3 |
| 68 | 2 | 157 | 3 |
| 73 | 2 | 158 | 2 |
| 78 | 2 | 160 | 2 |
| 89 | 2 | 205 | 3 |
| 92 | 3 | 206 | 2 |
| 93 | 3 | 208 | 3 |
| 95 | 2 | 210 | 3 |
| 108 | 2 | 213 | 3 |

Table 2, continued

Puccinia recondita test

256 ppm protective spray

| No. | Activity | No. | Activity |
|---|---|---|---|
| 214 | 3 | 291 | 3 |
| 215 | 2 | 292 | 3 |
| 219 | 2 | 293 | 3 |
| 220 | 2 | 294 | 3 |
| 222 | 3 | 295 | 3 |
| 223 | 2 | 297 | 3 |
| 228 | 2 | 301 | 3 |
| 231 | 2 | 306 | 3 |
| 233 | 2 | 308 | 3 |
| 238 | 2 | 312 | 3 |
| 241 | 3 | 313 | 3 |
| 242 | 3 | 1002 | 2 |
| 243 | 3 | 1003 | 2 |
| 248 | 2 | 1004 | 2 |
| 255 | 2 | 1006 | 2 |
| 256 | 3 | 1007 | 3 |
| 257 | 3 | 1010 | 2 |
| 259 | 3 | 1012 | 2 |
| 261 | 3 | 1013 | 2 |
| 262 | 3 | 1015 | 2 |
| 263 | 2 | 1028 | 3 |
| 264 | 3 | | |
| 273 | 3 | | |
| 276 | 3 | | |
| 277 | 2 | | |
| 278 | 2 | | |
| 279 | 3 | | |
| 284 | 3 | | |
| 286 | 2 | | |
| 289 | 3 | | |
| 290 | 3 | | |

Example 3

Botrytis leaf disc test, Cucumber, protective test.
Solvent: 10% acetone in water
Emulsifier: Triton X-155, 100 ppm

For preparing an appropriate composition of active substance the active substance is dissolved in the specified solvent with emulsifier to the concentration desired. Further dilution is carried out by a solution containing 10% acetone in water and 100 ppm Triton X-155.

In the test for protective activity cucumber leaf discs are soaked in the solution of the active compound for 1 min. and thereafter airdryed. Paperdiscs inoculated with Botrytis cinerea are placed on the center of the leaf discs.

Incubation is in a moist chamber on glass plates at 23° C.

Disease assessment is made after 2 - 3 days, (when untreated leaf discs are covered with mycelium). The activity of the compounds is shown in table 3. and is indicated as given below.

| | | |
|---|---|---|
| 3 complete | disease control | – no growth |
| 2 partiel | disease control | – growth only on the paperdisc |
| 1 partiel | disease control | – inhibited growth on the leaf disc |
| 0 no | disease control | – uninhibited growth on the leaf disc |

## Table 3

Botrytis leaf disc test.
Protective test, 512 ppm active substance.

| No. | Activity |
|---|---|
| 13 | 3 |
| 16 | 3 |
| 19 | 2 |
| 24 | 3 |
| 33 | 2 |
| 45 | 2 |
| 56 | 2 |
| 71 | 2 |
| 78 | 3 |
| 90 | 2 |
| 92 | 2 |
| 110 | 2 |
| 115 | 2 |
| 121 | 2 |
| 125 | 2 |
| 133 | 2 |
| 147 | 2 |
| 148 | 3 |
| 149 | 3 |
| 152 | 2 |
| 156 | 2 |
| 158 | 2 |
| 159 | 2 |
| 206 | 2 |
| 208 | 3 |
| 210 | 2 |
| 231 | 2 |
| 232 | 2 |
| 233 | 2 |
| 241 | 2 |

Example 4Agar plate test with :
Botrytis cinerea B.c.
Fusarium oxysporum F.o.
Pyricularia oryzae P.o
Pythium ultimum P.u.
Rhizoctonia solani R.s.
Septoria nodorum S.n.

Solvent: Acetone

The active compounds are dissolved in the specified solvent to the concentration desired.

The solutions of the active compounds are incorporated in agarplates to the concentrations 200, 20 and 2 ppm.

The plates are inoculated with the above mentioned organism and are incubated for 5 days at 23° C.

The activity of the compounds, which is shown in table 4., is assessed in comparison with the untreated agar plates and indicated as given below.

| | | |
|---|---|---|
| 3 complete | inhibition | - no growth |
| 2 partiel | inhibition | - growth only on the inoculation plug |
| 1 partiel | inhibition | - inhibited growth on the agar plates |
| - no | inhibition | - uninhibited growth on the agar plates |

Table 4

Agar plate test

| No. | B.c | F.o | P.o | P.u | R.s | S.n |
|---|---|---|---|---|---|---|
| 1 | 332 | 22- | 222 | 32- | 32- | 22- |
| 4 | 32- | 2-- | 32- | 2-- | 3-- | 2-- |
| 13 | 322 | 22- | 322 | 3-- | 32- | 22- |
| 16 | 32- | 22- | 222 | 3-- | 3-- | 2-- |
| 18 | 31- | 21- | 331 | 3-- | 31- | 21- |
| 19 | 32- | 21- | 321 | 3-- | 21- | 21- |
| 24 | 321 | 221 | 321 | 2-- | 32- | 21- |
| 26 | 31- | 21- | 31- | 3-- | 2-- | 1-- |
| 33 | 321 | 221 | 322 | 31- | 11- | 11- |
| 45 | 331 | 221 | 322 | 31- | 321 | 21- |
| 59 | 321 | 221 | 211 | 1-- | 12- | 11- |
| 60 | 321 | 21- | 321 | 3-- | 31- | 1-- |
| 61 | 31- | 21- | 211 | 3-- | 31- | 1-- |
| 62 | 321 | 21- | 321 | 2-- | 31- | 21- |
| 67 | 321 | 21- | 321 | 2-- | 31- | 21- |
| 68 | 32- | 21- | 211 | 2-- | 31- | 1-- |
| 71 | 321 | 221 | 321 | 3-- | 321 | 21- |
| 78 | 32- | 1-- | 321 | 3-- | 31- | 1-- |
| 92 | 32- | 21- | 321 | 3-- | 11- | 1-- |
| 93 | 31- | 21- | 221 | 3-- | 21- | 1-- |
| 95 | 32- | 21- | 321 | 3-- | 2-- | 2-- |
| 102 | 31- | 2-- | 321 | 3-- | 3-- | 2-- |
| 103 | 32- | 21- | 321 | 3-- | 3-- | 2-- |
| 104 | 3-- | 2-- | 311 | 3-- | 1-- | 1-- |
| 106 | 31- | 21- | 21- | 3-- | 3-- | 1-- |
| 107 | 31- | 2-- | 21- | 3-- | 3-- | 1-- |
| 108 | 21- | 21- | 21- | 3-- | 2-- | 2-- |
| 109 | 31- | 2-- | 31- | 3-- | 2-- | 1-- |
| 110 | 32- | 21- | 321 | 3-- | 2-- | 2-- |
| 111 | 321 | 22- | 32211 | 3-- | 31- | 21- |
| 112 | 321 | 221 | 221 | 2-- | 21- | 21- |
| 113 | 32- | 21- | 31- | 3-- | 2-- | 1-- |
| 118 | 321 | 211 | 221 | 31- | 321 | 21- |
| 119 | 321 | 21- | 321 | 31- | 31- | 21- |
| 120 | 332 | 321 | 322 | 31- | 321 | 21- |
| 121 | 321 | 21- | 221 | 3-- | 32- | 2*- |
| 122 | 32- | 21- | 3221- | 3-- | 2-- | 1-- |
| 123 | 32- | 2-- | 21- | 3-- | 3-- | 1-- |
| 124 | 32- | 21- | 21- | 3-- | 3-- | 1-- |
| 125 | 332 | 22- | 321-- | 3-- | 32- | 2-- |
| 127 | 332 | 221 | 331 | 31- | 22- | 21- |
| 130 | 321 | 21- | 321 | 31- | 21- | 1-- |
| 131 | 31- | 31- | 2221- | 3-- | 3-- | 2-- |
| 133 | 331 | 21- | 322 | 3-- | 3-- | 2-- |
| 134 | 33- | 21- | 321 | 3-- | 2-- | 2-- |
| 136 | 33- | 21- | 2221- | 31- | 2-- | 21- |
| 138 | 321 | 2-- | 321 | 2-- | 21- | 2-- |
| 139 | 321 | 2-- | 322 | 2-- | 21- | 2-- |

Table 4, continued

Agar plate test

| No. | B.c | F.o | P.o | P.u | R.s | S.n |
|---|---|---|---|---|---|---|
| 141 | 332 | 22- | 332 | 32- | 31- | 21- |
| 142 | 332 | 22- | 3321- | 32- | 22- | 2-- |
| 143 | 332 | 22- | 321-- | 3-- | 22- | 1-- |
| 147 | 32- | 2-- | 21- | 3-- | 3-- | 1-- |
| 148 | 321 | 2-- | 221 | 3-- | 3-- | 2-- |
| 149 | 32- | 2-- | 321 | 3-- | 3-- | 2-- |
| 150 | 331 | 21- | 321 | 3-- | 31- | 2-- |
| 151 | 331 | 31- | 221 | 3-- | 31- | 21- |
| 152 | 31- | 2-- | 31- | 3-- | 2-- | 2-- |
| 153 | 32- | 21- | 32- | 3-- | 3-- | 2-- |
| 154 | 332 | 21- | 321 | 3-- | 31- | 21- |
| 155 | 331 | 21- | 22- | 31- | 31- | 2-- |
| 156 | 32- | 21- | 32- | 3-- | 3-- | 2-- |
| 157 | 322 | 321 | 221 | 31- | 211 | 211 |
| 158 | 322 | 221 | 2222- | 3-- | 31- | 21- |
| 204 | 332 | 21- | 321-- | 2-- | 21- | 21- |
| 205 | 31- | 21- | 31- | 3-- | 2-- | 1-- |
| 206 | 32- | 21- | 321 | 3-- | 2-- | 1-- |
| 207 | 31- | 2-- | 31- | 3-- | 2-- | 1-- |
| 208 | 32- | 21- | 321 | 3-- | 3-- | 1-- |
| 209 | 32- | 2-- | 321 | 3-- | 2-- | 1-- |
| 210 | 331 | 2-- | 321 | 3-- | 3-- | 2-- |
| 211 | 332 | 22- | 321-- | 32- | 321 | 211 |
| 212 | 31- | 2-- | 321 | 3-- | 2-- | 1-- |
| 213 | 332 | 2-- | 321 | 3-- | 31- | 21- |
| 214 | 33- | 2-- | 321 | 3-- | 21- | 2-- |
| 215 | 331 | 21- | 221 | 3-- | 21- | 2-- |
| 217 | 32- | 21- | 321 | 3-- | 3-- | 2-- |
| 218 | 32- | 21- | 321 | 3-- | 31- | 2-- |
| 219 | 321 | 21- | 221 | 2-- | 331 | 21- |
| 220 | 321 | 21- | 321 | 3-- | 21- | 211 |
| 221 | 32- | 21- | 321 | 3-- | 31- | 21- |
| 222 | 32- | 21- | 321 | 3-- | 211 | 21- |
| 223 | 321 | 21- | 321 | 3-- | 22- | 21- |
| 227 | 332 | 221 | 332 | 31- | 321 | 211 |
| 228 | 331 | 211 | 321 | 32- | 321 | 211 |
| 231 | 321 | 221 | 321 | 31- | 32- | 21- |
| 233 | 321 | 221 | 332 | 21- | 32- | 21- |
| 234 | 322 | 221 | 221 | 2-- | 32- | 21- |
| 237 | 322 | 22- | 221 | 32- | 21- | 211 |
| 241 | 321 | 21- | 321 | 3-- | 31- | 21- |
| 242 | 332 | 31- | 321 | 31- | 31- | 311 |
| 243 | 331 | 31- | 221 | 3-- | 31- | 2-- |
| 248 | 322 | 221 | 222 | 21- | 22- | 211 |
| 249 | 321 | 21- | 221 | 21- | 331 | 211 |
| 250 | 3-- | 2-- | 321 | 3-- | 2-- | 1-- |
| 252 | 331 | 221 | 322 | 32- | 22- | 21- |
| 255 | 32- | 2-- | 321 | 3-- | 31- | 21- |
| 256 | 322 | 21- | 332 | 3-- | 321 | 21- |

Table 4, continued

Agar plate test

| No. | B.c | F.o | P.o | P.u | R.s | S.n |
|---|---|---|---|---|---|---|
| 257 | 32- | 21- | 321 | 3-- | 21- | 21- |
| 259 | 321 | 2-- | 22- | 3-.- | 3-- | 2-- |
| 260 | 33- | 21- | 22- | 3-- | 2-- | 2-- |
| 261 | 321 | 21- | 221 | 31- | 21- | 211 |
| 262 | 322 | 21- | 321 | 21- | 21- | 21- |
| 263 | 33- | 2-- | 321 | 3-- | 2-- | 1-- |
| 264 | 332 | 2-- | 321 | 3-- | 21- | 2-- |
| 265 | 32- | 3-- | 321 | 3-- | 2-- | 2-- |
| 284 | 321 | 21- | 321 | 3-- | 31- | 1-- |
| 286 | 332 | 221 | 322 | 33- | 21- | 211 |
| 288 | 321 | 21- | 321 | 31- | 21- | 2-- |
| 296 | 332 | 211 | 221 | 32- | 211 | 21- |

## Claims

1. Amino-alkylated hydroxy compounds, characterised by having the general formula I

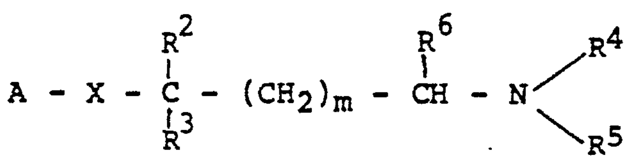

I

wherein A represents

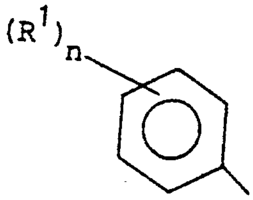

or

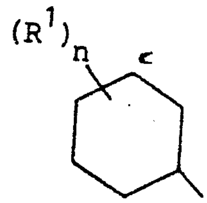

wherein $(R^1)_n$ represents up to four substituents which are the same or different and selected from straight or branched chain, substituted or unsubstituted alkyl having up to 12 carbon atoms, aryl, aryloxy, alkoxy, arylalkyl, haloalkyl, dialkylamino, halogen, alkylthio and nitro, where two adjacent $R^1$-substituents may also together form a saturated or unsaturated ring, and n is 0, 1, 2, 3 or 4,
$R^2$ and $R^3$ each independently represents hydrogen or straight or branched chain alkyl having up to 6 carbon atoms,
$R^4$ and $R^5$ each independently represents hydrogen, straight or branched chain or cyclic, substituted or unsubstituted alkyl having up to 12 carbon atoms, alkenyl, alkynyl dsor sustituted or unsubstituted arylalkyl, or $R^4$ and $R^5$ when taken together with the nitrogen atom to which they are attached form a saturated or unsaturated, substituted, unsubstituted, aryl-fused or cycloalkyl-fused heterocyclic ring which may optionally contain one or more additional hetero atoms selected from 0 and N,
$R^6$ represents hydrogen or lower alkyl,

X represents 0 or S, and
m is 0, 1 or 2 when A represents substituted or unsubstituted phenyl, and m is 0 or 1 when A represents substituted or unsubstituted cyclohexyl, and quaternary ammonium salts thereof, preferably prepared by reaction with alkyl, alkenyl, arylalkyl or alkoxycarbonylmethyl halides.

2. A fungicidal compositin, particularly a plant-fungicidal composition, characterised by containing as an active component at least one compound selected from the amino-alkylated hydroxy compounds of formula I as defined in claim 1 and quaternary ammonium salts thereof.

3. Use of an amino-alkylated hydroxy compound of formula I as defined in claim 1 and/or a quaternary ammonium salt thereof for combating fungi, particularly phytopathogenic fungi.

4. A method of combating fungi, particularly phytopathogenic fungi, characterised by allowing an amino-alkylated hydroxy compound of formula I as defined in claim 1 and/or a quaternary ammonium salt thereof to affect the fungi concerned and/or their biotope.

5. A process of preparing a fungicidal composition, particularly a plant-fungicidal composition, characterised by mixing an amino-alkylated hydroxy compound of formula I as defined in claim 1 and/or a quaternary ammonium salt thereof with one or more agents selected from extending agents, surface active agents and other conventional auxiliary agents.